# EUROPEAN PATENT SPECIFICATION

(11) **EP 1 475 401 B1**
(45) Date of publication and mention of the grant of the patent: **27.05.2009**
(21) Application number: 04002898.7
(22) Date of filing: 10.02.2004
(51) Int. Cl.: C08G 61/08, C09K 11/06, C07D 333/16, C08G 61/02, C08G 61/12, C07D 333/08, C07D 333/12

(54) **Mono-, oligo- and polymers comprising fluorene and aryl groups**
Fluorene und Arylgruppen enthaltende Mono-, Oligo- und Polymere
Monomères, oligomères et polymères comprenant des groupes fluorène et aryle.

(30) Priority: 07.03.2003 EP 03004925
(43) Date of publication of application: 10.11.2004
(73) Proprietor: Merck Patent GmbH, 64293 Darmstadt (DE)
(72) Inventor: Heeney, Martin, Southampton SO14 6TQ (GB); Tierney, Steven, Southampton SO15 7QW (GB); Thomson, Marcus, Mudeford Christchurch BH23 3JR Dorset (GB); McCulloch, Iain, Dr., Chandlers Ford Southampton SO53 4LG (GB)

(56) References cited:
- EP-A- 1 323 762
- WO-A-00/46321
- US-B1- 6 495 273
- TIRAPATTUR ET AL.: "Spectroscopic Study of Intermolecular Interactions in Various Oligofluorenes: Precursors of Light-Emitting Polymers" JOURNAL OF PHYSICAL CHEMISTRY, vol. 106, 2002, pages 8959-8966, XP002321534
- ASAWAPIROM ET AL: 'Dialkylfluoerene-Oligothiophene and Dialkylfluorene-Dithienylvinylene Alternating Copolymers' SYNTHESIS vol. 9, 2002, pages 1136 - 1142, XP001537816
- MCKENN ET AL: 'Stucture-property relationship for alpha, omega-dialkyl substituted thiphene/fluorene based oligomers' POLYMER PREPRINTS vol. 44, no. 2, 2003, page 441

## Description

### Field of Invention

The invention relates to mono-, oligo- or polymers, in particular conjugated copolymers, comprising 9-H,H-fluorene and aryl groups. The invention further relates to methods of their preparation, to their use as semiconductors or charge transport materials in optical, electrooptical or electronic devices including field effect transistors, electroluminescent, photovoltaic and sensor devices. The invention further relates to field effect transistors and semiconducting components comprising the new materials.

### Background and Prior Art

Organic materials have recently shown promise as the active layer in organic based thin film transistors and organic field effect transistors [see H. E. Katz et al., *Acc. Chem.* Res., 2001, **34**, 5, p.359]. Such devices have potential applications in smart cards, security tags and the switching element in flat panel displays. Organic materials are envisaged to have substantial cost advantages over their silicon analogues if they can be deposited from solution, as this enables a fast, large-area fabrication route.

The performance of the device is principally based upon the charge carrier mobility of the semiconducting material and the current on/off ratio, so the ideal semiconductor should have a low conductivity in the off state, combined with a high charge carrier mobility (> 1 x 10⁻³ cm² V⁻¹ S⁻¹). In addition, it is important that the semiconducting material is relatively stable to oxidation i.e. it has a high ionisation potential, as oxidation leads to reduced device performance.

Compounds known in prior art which have been shown to be effective p-type semiconductors for organic FETs are dithienohiophene (DTT) (**1a**) and its fused dimer α,α'-bis(dithieno 3,2-b:2',3'-d]thiophene (BDT) (1b) having the structures shown below [see H. Sirringhaus et al., Appl. Phys. Lett., 1997, 71, 26, p.3871; Li et al., J. Am. Chem. Soc., 1998, 120, p.2206; J. J. Morrison et al., Synth. Met., 1999, 102, p.987].

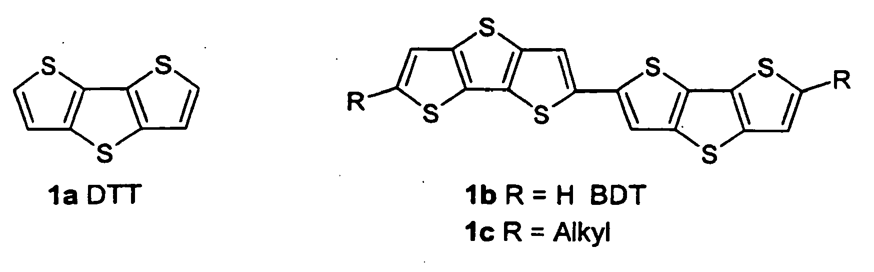

In particular BDT, which has been extensively studied, has been shown to be an effective p-type semiconductor for organic FETs with a very high charge carrier mobility between 1 x 10⁻³ and 5 x 10⁻² cm² V⁻¹ s⁻¹ and very high current on/off ratios (up to 10⁸). BDT also has been found in the solid state to have a completely coplanar formation, and to be more planar than oligomers of thiophene.

However, BDT has a high melting point and is very insoluble, therefore, if used as the active layer in an organic thin film transistor, it cannot be readily solution processed. As a result, for applications like FETs, prior art materials like BDT are usually deposited as a thin film by vacuum deposition, which is an expensive processing technique that is unsuitable for the fabrication of large-area films. To improve the solubility of BDT, several substituted derivatives have so far been synthesized **(1c),** [see J. J. Morrison et al., Synth. Met., 1999, 102, p.987] but these have still required vacuum processing when used in thin film transistors.

It is the aim of the present invention to provide new materials for use as semiconductors or charge transport materials, which are easy to synthesize, have high charge mobility and are easily processible to form thin and large-area films for use in semiconductor devices. Other aims of the invention are immediately evident to those skilled in the art from the following description.

The inventors have found that these aims can be achieved by providing new aryl copolymers of 9-H,H-fluorene.

9,9-Dialkylfluorene (**2**) containing (co)polymers have been extensively investigated as a class of soluble material for organic light emitting diode (OLED) applications [see M.T. Bernius et al., Adv. Mater., 2000, 12(23), p.1737; U. Scherf et al., Adv. Mater., 2002, 14(7), p.477]. For OLED applications it is desirable to prevent aggregation of the polymer backbones which can lead to excimer quenching. The 9,9-dialkyl substituents help to provide solubility for the polymer and also pack orthogonal to the polymer backbone, thus helping to prevent aggregation of the polymer chains.

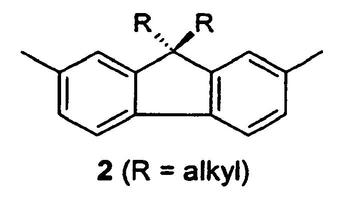

US 5,708,130 and US 6,169,163 disclose polymers of 9,9'-disubstituted fluorene as shown in structure (**3**), wherein R is C₁₋₂₀-hydrocarbyl that may also contain hetero atoms. WO 00/22026 discloses polymers of 9,9'-disubstituted fluorene wherein R is aryl or heteroaryl. US 6,353,083 discloses copolymers of 9-substituted or 9,9'-disubstituted fluorene with two other different monomers. These documents further suggest to use the polymers for OLEDs.

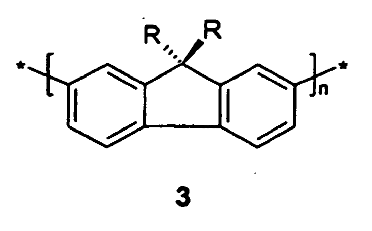

Bin Liu et al., Macromolecules, 2000, 33, p.8945 report the synthesis of copolymers of 9,9'-dihexylfluorene and decylthiophene and the investigation of these materials for OLED applications.

WO 02/45184 discloses a field effect transistor (FET) comprising an organic semiconductor and a binder, wherein the semiconductor may inter alia comprise polymers of structure (**3**) above, wherein R is selected from H, alkyl, aryl or substituted aryl.

S. Tirapattur et al., J. Phys. Chem. B 2002, 106, 8959-8966, EP 1 323 762 A2 and McKenn et al., Polymer Preprints 2003, 44(2), 441 do also disclose mono-, oligo- and polyfluorenes, but do not disclose compounds as claimed in the present invention.

H. Sirringhaus et al., Appl. Phys. Lett., 2000, 77(3), p.406 report poly-9,9-dioctyl-fluorene-co-bithiophene (**4**, F8T2) to show reasonable mobility (10⁻² cm²V⁻¹s⁻¹) in a field effect transistor despite the presence of octyl chains on the sp³ -carbon of the fluorene bridge. This good mobility is a consequence of the liquid crystalline behaviour of the polymer, which allows control of the morphology of the semiconductor in the transistor. Specifically the polymer is annealed in ints nematic liquid crystal phase at 275 - 285 °C and aligned on a rubbed polyimide layer in the direction of charge transport before quenching to 'freeze in' the order.

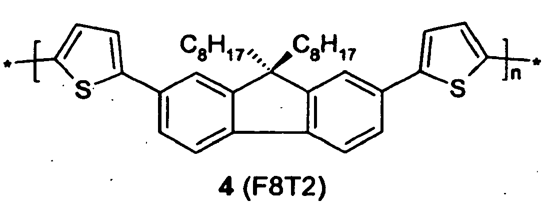

However, fluorene co-polymers of prior art which are substituted at the sp³ carbon atom in 9-position with hydrocarbon groups have the disadvantage that the sp³ dialkyl bridge disrupts close packing, which negatively affects charge transport.

In contrast, the present invention relates to unsubstituted fluorene co-polymers which are not substituted at the sp³ carbon in 9-position of the fluorene bridge but retain mesogenic behaviour. The absence of chains at the fluorene bridge facilitates closer packing of the polymer backbones and improved charge transport. The necessary solubilising side-chains are now present on the thiophene comonomer and are able to lie in the plane of the polymer backbone, therefore not disrupting the packing of polymer chains. Furthermore this approach affords polymers which exhibit liquid crystalline behaviour at lower temperatures. Thus, some of the materials of the present invention exhibit liquid crystallinity already at temperatures around 150 °C, which is considerably lower than for example the polymer F8T2 of prior art, which has a nematic phase at a temperature of 265 °C or higher, and enables lower processing temperatures e.g. in the fabrication of FET devices.

A further aspect of the invention relates to reactive mesogens having a central core comprising 9-H,H-fluorene and arylene units, said core being linked, optionally via a spacer group, to one or two polymerisable groups. The reactive mesogens can induce or enhance liquid crystal phases or are liquid crystalline themselves. They can be oriented in their mesophase and the polymerisable group(s) can be polymerised or crosslinked in situ to form polymer films with a high degree of order, thus yielding improved semiconductor materials with high stability and high charge carrier mobility.

A further aspect of the invention relates to liquid crystal polymers like liquid crystal main chain or side chain polymers, in particular liquid crystal side chain polymers obtained from the reactive mesogens according to the present invention, which are then further processed, e.g., from solution as thin layers for use in semiconductor devices.

### Summary of the Invention

The invention relates to mono-, oligo- or polymers as claimed in claim 1.

The invention further relates to a semiconductor or charge transport material, component or device comprising at least one mono-, oligo- or polymer as defined above.

The invention further relates to the use of polymers according to the invention as semiconductors or charge transport materials, in particular in optical, electrooptical or electronic devices, like for example in field effect transistors (FET) as components of integrated circuitry, as thin film transistors in flat panel display applications or for Radio Frequency Identification (RFID) tags, or in semiconducting components for organic light emitting diode (OLED) applications such as electroluminescent displays or backlights of e.g. liquid crystal displays (LCD), for photovoltaic or sensor devices, as electrode materials in batteries, as photoconductors and for electrophotographic applications like electrophotographic recording.

The invention further relates to the use of the novel mono-, oligo- and polymers according to the present invention as electroluminescent materials, in photovoltaic or sensor devices, as electrode materials in batteries, as photoconductors, for electrophotographic applications like electrophotographic recording and as alignment layer in LCD or OLED devices.

The invention further relates to an optical, electrooptical or electronic device, FET, integrated circuit (IC), TFT, OLED or alignment layer comprising a semiconducting or charge transport material, component or device according to the invention.

The invention further relates to a TFT or TFT array for flat panel displays, radio frequency identification (RFID) tag, electroluminescent display or backlight comprising a semiconducting or charge transport material, component or device or a FET, IC, TFT or OLED according to the invention.

The invention further relates to a security marking or device comprising a FET or an RFID tag according to the invention.

### Detailed Description of the Invention

The mono-, oligo and polymers according to the invention are especially useful as charge transport semiconductors in that they halve high carrier mobilities.

In the mono-, oligo and polymers according to the present invention the 9-H,H-fluorene group can be unsubstituted or substituted in other than the 9-position. The arylene group can be substituted or unsubstituted and optionally comprises one or more hetero atoms.

In a preferred embodiment of the present invention the 9-H,H-fluorene group is substituted in other than 9-position by one or more alkyl or fluoroalkyl groups. The introduction of fluoroalkyl and alkyl side chains into the 9-H,H-fluorene group improves their solubility and therefore their solution processibility. Furthermore, the presence of fluoroalkyl side chains also renders the materials of the present invention effective as *n*-type semiconductors. The electron-withdrawing nature of the fluoroalkyl substituents will also lower the HOMO (highest occupied molecular orbital energy level) further and result in a more stable material, which is less susceptible to oxidation.

The mono-, oligo-, and polymers have one or more identical or different recurring units of formula I 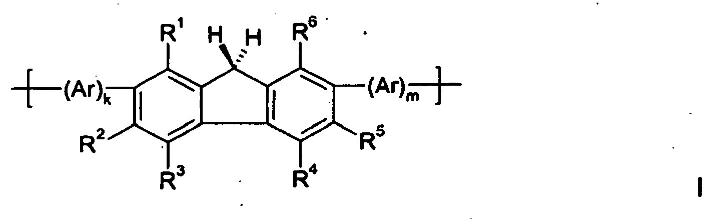 wherein
- R¹ to R⁶: are independently of each other H, halogen or straight chain, branched or cyclic alkyl with 1 to 20 C-atoms, which may be unsubstituted, mono- or poly-substituted by F, Cl, Br, I and/or CN, it being also possible for one or more non-adjacent CH₂ groups to be replaced, in each case independently from one another, by -O-, -S-, -NH-,-NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another, optionally substituted aryl or heteroaryl, or P-Sp-,
- P: is a polymerisable or reactive group,
- Sp: is a spacer group or a single bond,
- R⁰ and R⁰⁰: are independently of each other H or alkyl with 1 to 12 C-atoms, and
- Ar: is an arylene or heteroarylene group that is optionally substituted with one or more groups R¹, and
- k and m: are independently of each other 0, 1, 2, 3 or 4, with k+m > 1, or k is 0 or 1 and m is 1 or 2.

The mono-, oligo-, and polymers are of formula I1 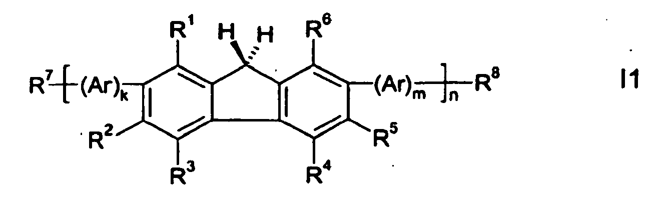 wherein R¹⁻⁶, Ar, k and m have independently of each other one of the meanings of formula I,
- R⁷ and R⁸: are independently of each other H, halogen, Sn(R⁰)₃ or straight chain, branched or cyclic alkyl with 1 to 20 C-atoms, which may be unsubstituted, mono- or poly-substituted by F, Cl, Br, I, -CN and/ or -OH, it being also possible for one or more non-adjacent CH₂ groups to be replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another, optionally substituted aryl or heteroaryl, or P-Sp,
- n: is an integer from 1 to 10000,
and wherein the recurring units may be identical or different,
wherein
n is from 2 to 5000, or
n is an integer from 1 to 15 and one or both of R⁷ and R⁸ denote P-Sp-, or
R⁷ and R⁸ are selected from halogen, Sn(R⁰)₃ , CH₂Cl, COH, CH=CH₂, SiR⁰R⁰⁰.

The recurring units of formula I are hereinafter also abbreviated as [(Ar)ₖ-fl-(Ar)ₘ] wherein fl is a 9-H,H-fluorene unit as shown in formula I and Ar, k and m are as defined in formula I.

In the oligo- and polymers of the present invention the recurring units [(Ar)ₖ-fl-(Ar)ₘ] in case of multiple occurrence can be selected of formula I independently of each other, so that an oligo- or polymer may comprise identical or different recurring units [(Ar)ₖ-fl-(Ar)ₘ]. The oligo- and polymers thus include homopolymers and copolymers like for example
- statistically random copolymers, for example with a monomer sequence such as -fl-Ar-Ar-fl-fl-fl-Ar-fl-Ar-,
- alternating copolymers with the monomer sequence -fl-Ar-fl-Ar-fl-Ar-fl-Ar-, and
- block copolymers, for example with a monomer sequence such as -fl-fl-fl-fl-fl-Ar-Ar-Ar-Ar-Ar-Ar-fl-fl-fl-fl-fl-fl-,
wherein the groups fl and Ar preferably together form a conjugated system, and wherein multiple groups (for example each group fl in the sequence -fl-fl-fl-fl-, or each group Ar in the sequence -Ar-Ar-Ar-Ar-Ar-Ar-) can be identical or different from one another.

Especially preferred are mono-, oligo- and polymers comprising one or more recurring units [(Ar)ₖ-fl-(Ar)ₘ], wherein k is 0, m is 1, all groups fl have the same meaning and all groups Ar have the same meaning, very preferably mono-, oligo- and polymers consisting exclusively of such recurring units.

Further preferred are oligo-, and polymers of formula I and I1 having identical recurring units.

Further preferred are mono-, oligo-, and polymers of formula I and I1 wherein R¹⁻⁶ are H.

Especially preferred are oligo-, and polymers of formula I and I1 having a degree of polymerisation (number n of recurring units) from 2 to 5,000, in particular from 10 to 5,000, very preferably from 100 to 1,000.

Further preferred are oligo-, and polymers of formula I and I1 having a molecular weight from 5,000 to 300,000, in particular from 20,000 to 100,000.

Especially preferred are regioregular polymers of formula I and I1. The regioregularity in these polymers is preferably at least 90 %, in particular 95% or more, very preferably 98% or more, most preferably from 99 to 100%.

Regioregular polymers are advantageous as they show strong interchain pi-pi-stacking interactions and a high degree of crystallinity, making them effective charge transport materials with high carrier mobilities.

Further preferred are mono-, oligo- and polymers of formula I and I1 comprising at least one reactive group P that is capable of a polymerisation or crosslinking reaction.

Further preferred are mono-, oligo- and polymers of formula I and I1 that are mesogenic or liquid crystalline, in particular polymers forming calamitic phases, and reactive mesogens of formula I or I1 comprising one or more groups P-Sp-, forming calamitic phases.

Further preferred are mono-, oligo- and polymers of formula I and I1 shown above and below wherein
- m is 1, 2 or 3, preferably 1 or 2,
- k is 0, 1 or 2, preferably 0 or 1,
- n is an integer from 1 to 15, preferably 1, and one or both of R⁷ and R⁸ denote P-Sp-,
- at least one of R¹⁻⁶ denotes P-Sp-,
- n is an integer from 2 to 5000 and R⁷ and R⁸ are different from P-Sp-,
- R¹ to R⁶ are selected from C₁-C₂₀-alkyl that is optionally substituted with one or more fluorine atoms, C₁-C₂₀-alkenyl, C₁-C₂₀-alkynyl, C₁-C₂₀-alkoxy, C₁-C₂₀-thioalkyl, C₁-C₂₀-silyl, C₁-C₂₀-ester, C₁-C₂₀-amino, C₁-C₂₀-fluoroalkyl, (CH₂CH₂O)ₘCᵢH₂ᵢ₊₁ with m being an integer from 1 to 6 and i being 1, 2 or 3, and optionally substituted aryl or heteroaryl, very preferably C₁-C₂₀-alkyl or C₁-C₂₀-fluoroalkyl,
- R¹, R⁷ and R⁸ are selected from H, halogen, Sn(R⁰)₃ , CH₂Cl, COH, CH=CH₂, SiR⁰R⁰⁰ and optionally substituted aryl or heteroaryl,
- n > 1.

Ar is preferably a mono-, bi- or tricyclic aromatic or heteroaromatic group with up to 25 C atoms, wherein the rings can be fused, and in which the heteroaromatic group contains at least one hetero ring atom, preferably selected from N, O and S. It is optionally substituted with one or more of F, Cl, Br, I, CN, and straight chain, branched or cyclic alkyl having 1 to 20 C atoms, which is unsubstituted, mono- or poly-substituted by F, Cl, Br, I, -CN or -OH, and in which one or more non-adjacent CH₂ groups are optionally replaced, in each case such a manner that O and/or S atoms are not linked directly to one another.

Preferred groups Ar are phenyl, fluorinated phenyl, pyridine, pyrimidine, biphenyl, naphthalene, thiophene, fluorinated thiophene, benzo[1,2-b:4,5-b']dithiophene, cyclopenta[2,1-b:3,4-b']dithiophene, 9-alkylidenefluorene, 9-H,H-fluorene, thiazole and oxazole, all of which are unsubstituted, mono- or polysubstituted with R¹ as defined above, preferably with L, wherein L is F, Cl, Br, or an alkyl, alkoxy, alkylcarbonyl or alkoxycarbonyl group with 1 to 12 C atoms, wherein one or more H atoms are optionally replaced by F or Cl.

Very preferably Ar is thiophene-2,5-diyl that is optionally substituted in 3- or 4-position by R¹ or L. R¹ in these preferred groups is preferably C₁-C₂₀-alkyl that is optionally substituted with one or more fluorine atoms, C₁-C₂₀-alkenyl, C₁-C₂₀-alkynyl, C₁-C₂₀-alkoxy, C₁-C₂₀- thioalkyl, C₁-C₂₀-silyl, C₁-C₂₀-ester, C₁-C₂₀-amino, C₁-C₂₀-fluoroalkyl, (CH₂CH₂O)ₘ with m being an integer from 1 to 6, and optionally substituted aryl or heteroaryl, very preferably C₁-C₂₀-alkyl or C₁-C₂₀- fluoroalkyl.

Especially preferred are compounds of the following formulae 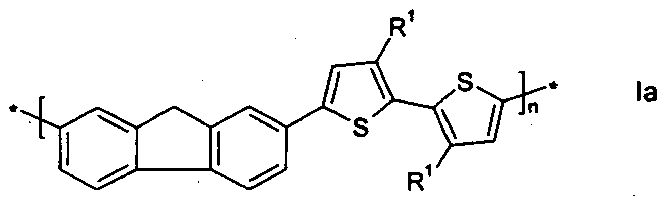 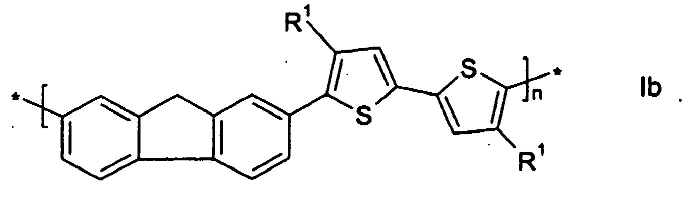 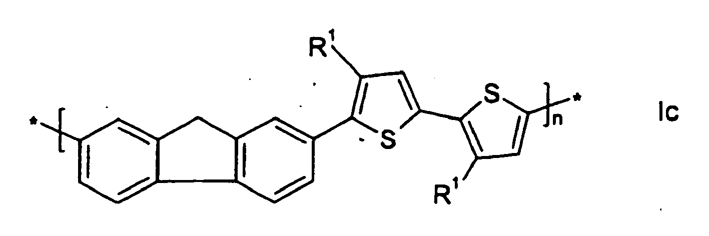 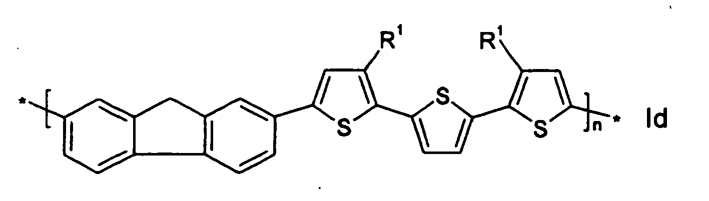 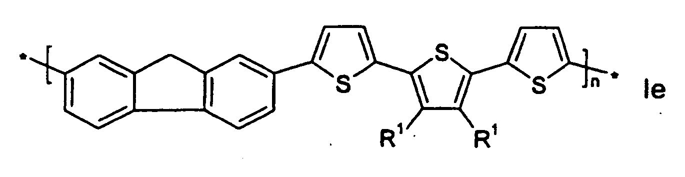 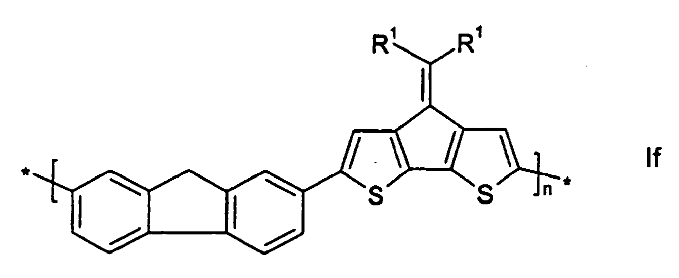 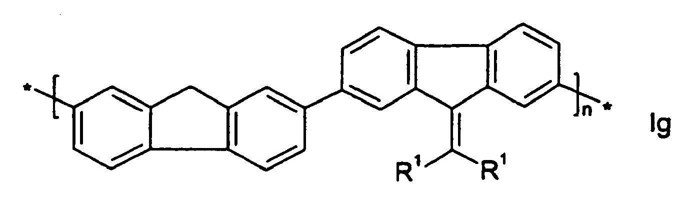 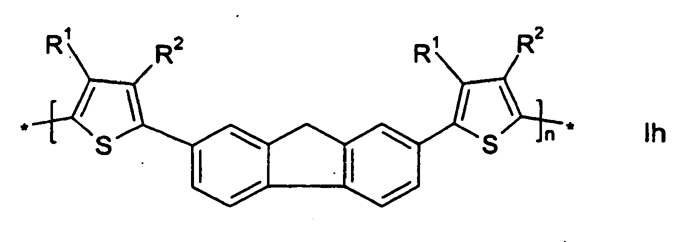 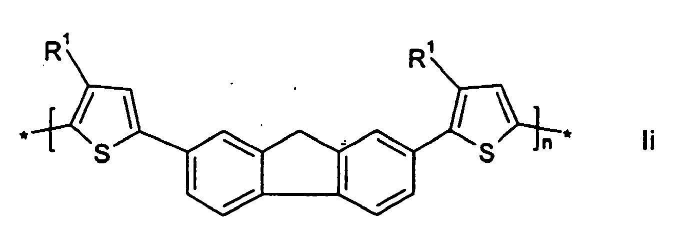 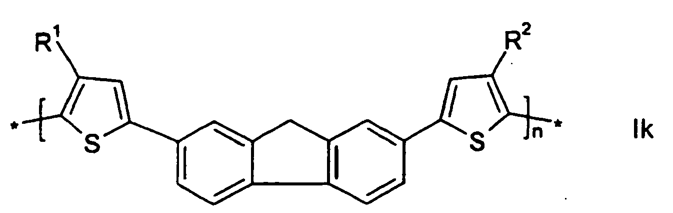 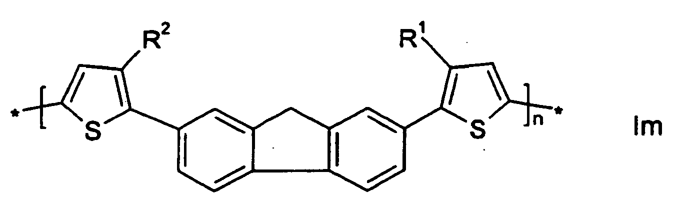 wherein R¹, R² and n are as defined above.

Further preferred are monomers of the following formulae 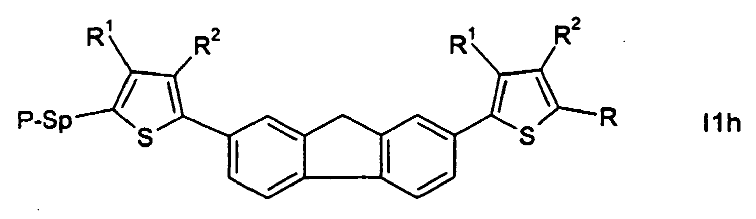 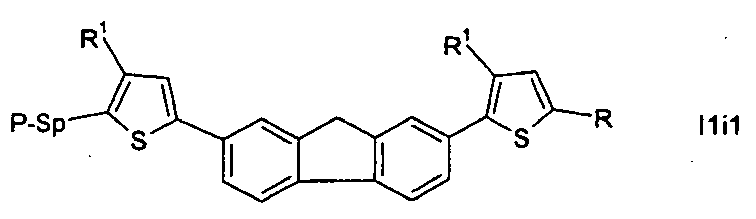 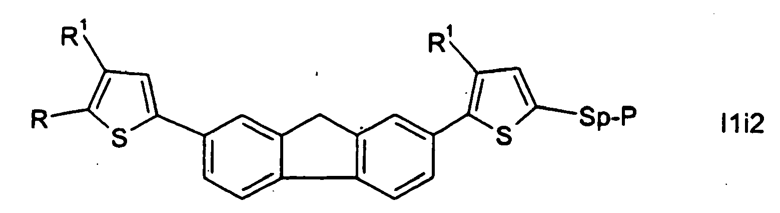 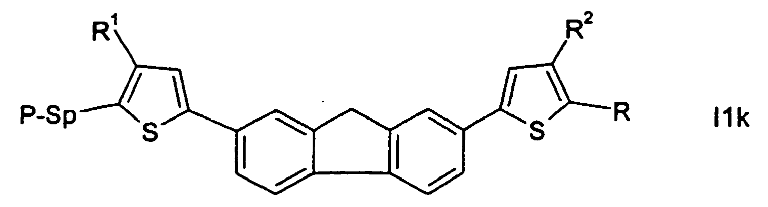 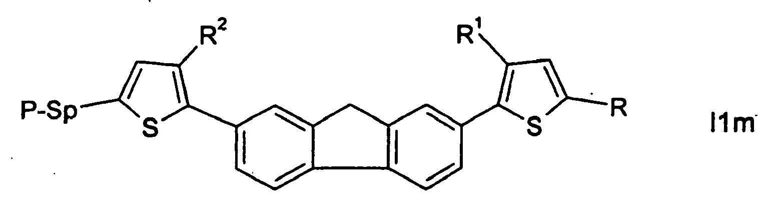 wherein P, Sp, R¹ and R² are as defined above and R has one of the meanings of R⁷. Preferably R is P-Sp-. Further preferably R¹ and R² are H. If R is P-Sp, the two groups P or the two groups Sp, respectively, can be identical or different.

If one of R¹⁻⁸ is aryl or heteroaryl, it is preferably a mono-, bi- or tricyclic aromatic or heteroaromatic group with up to 25 C atoms, wherein the rings can be fused, and in which the heteroaromatic group contains at least one hetero ring atom, preferably selected from N, O and S. It is optionally substituted with one or more of F, Cl, Br, I, CN, and straight chain, branched or cyclic alkyl having 1 to 20 C atoms, which is unsubstituted, mono- or poly-substituted by F, Cl, Br, I, -CN or -OH, and in which one or more non-adjacent CH₂ groups are optionally replaced, in each case independently from one another, by -O-, -S-, -NH-, -NR⁰-, -SiR⁰R⁰⁰-, -CO-, -COO-, OCO-, --OCO-O, -S-CO-, -CO-S-,-CH=CH- or -C≡C- in such a manner that O and/or S atoms are not linked directly to one another.

Especially preferred aryl and heteroaryl groups are phenyl, fluorinated phenyl, pyridine, pyrimidine, biphenyl, naphthalene, thiophene, fluorinated thiophene, benzo[1,2-b:4,5-b']dithiophene, thiazole and oxazole, all of which are unsubstituted, mono- or polysubstituted with L as defined above.

If one of R¹ to R⁸ is an alkyl or alkoxy radical, i.e. where the terminal CH₂ group is replaced by -O-, this may be straight-chain or branched. It is preferably straight-chain, has 2 to 8 carbon atoms and accordingly is preferably ethyl, propyl, butyl, pentyl, hexyl, heptyl, octyl, ethoxy, propoxy, butoxy, pentoxy, hexyloxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

Oxaalkyl, i.e. where one CH₂ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

Fluoroalkyl or fluorinated alkyl or alkoxy is preferably straight chain (O)CᵢF₂ᵢ₊₁, wherein i is an integer from 1 to 20, in particular from 1 to 15, very preferably (O)CF₃, (O)C₂F₅, (O)C₃F₇, (O)C₄F₉, (O)C₅F₁₁, (O)C₆F₁₃, (O)C₇F₁₅ or (O)C₈F₁₇, most preferably (O)C₆F₁₃.

Halogen is preferably F, Br or Cl.

Hetero atoms are preferably selected from N, O and S.

The polymerisable or reactive group P is preferably selected from

CH₂=CW¹-COO-, 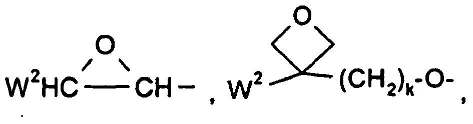 CH₂=CW²- (O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN-, and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene that is optionally substituted by one or more groups R¹ as defined above, and k₁ and k₂ being independently of each other 0 or 1.

Especially preferred groups P are CH₂=CH-COO-, CH₂=C(CH₃)-COO-, CH₂=CH-, CH₂=CH-O-, (CH₂=CH)₂CH-OCO-, (CH₂=CH)₂CH-O-, (CH₂=CH-CH₂)₂N-, (CH₂=CH-CH₂)₂N-CO- and

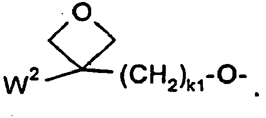

Very preferred are acrylate and oxetane groups. Oxetanes produce less shrinkage upon polymerisation (cross-linking), which results in less stress development within films, leading to higher retention of ordering and fewer defects. Oxetane cross-linking also requires cationic initiator, which unlike free radical initiator is inert to oxygen.

As for the spacer group Sp all groups can be used that are known for this purpose to the skilled in the art. The spacer group Sp is preferably of formula Sp'-X, such that P-Sp- is P-Sp'-X-, wherein
- Sp': is alkylene with up to 20 C atoms which may be unsubstituted, mono- or poly-substituted by F, Cl, Br, I or CN, it being also possible for one or more non-adjacent CH₂ groups to be replaced, in each case independently from one another, by -O-, - S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another,
- X: is -O-, -S-, -CO-, -COO-, -OCO-, -O-COO-, -CO-NR⁰-, -NR⁰-CO- , -OCH₂-, -CH₂O-, -SCH₂-, -CH₂S-, -CF₂O- -OCF₂-, -CF₂S-,-SCF₂-, -CF₂CH₂-, -CH₂CF₂-, -CF₂CF₂-, -CH=N-, -N=CH-, -N=N-, -CH=CR⁰-, -CX¹=CX²-, -C≡C-, -CH=CH-COO-, -OCO-CH=CH- or a single bond, and
- R⁰, R⁰⁰, X¹ and X²: have one of the meanings given above.

X is preferably -O-, -S-, -OCH₂-, -CH₂O- -SCH₂-, -CH₂S-, -CF₂O-,-OCF₂-, -CF₂S-, -SCF₂-, -CH₂CH₂-, -CF₂CH₂-, -CH₂CF₂-, -CF₂CF₂-,-CH=N-, -N=CH-, -N=N-, -CH=CR⁰-, -CX¹=CX²-, -C≡C- or a single bond, in particular -O-, -S-, -C≡C-, -CX¹=CX²- or a single bond, very preferably a group that is able to from a conjugated system, such as - C=C- or -CX¹=CX²-, or a single bond.

Typical groups Sp' are, for example, -(CH₂)ₚ-, -(CH₂CH₂O)_{q} -CH₂CH₂-,-CH₂CH₂-S-CH₂CH₂- or -CH₂CH₂-NH-CH₂CH₂- or -(SiR⁰R⁰⁰-O)ₚ-, with p being an integer from 2 to 12, q being an integer from 1 to 3 and R⁰ and R⁰⁰ having the meanings given above.

Preferred groups Sp' are ethylene, propylene, butylene, pentylene, hexylene, heptylene, octylene, nonylene, decylene, undecylene, dodecylene, octadecylene, ethyleneoxyethylene, methyleneoxybutylene, ethylene-thioethylene, ethylene-N-methyl-iminoethylene, 1-methylalkylene, ethenylene, propenylene and butenylene for example.

Further preferred are compounds with one or two groups P-Sp- wherein Sp is a single bond.

In case of compounds with two groups P-Sp, each of the two polymerisable groups P and the two spacer groups Sp can be identical or different.

SCLCPs obtained from the inventive compounds or mixtures by polymerisation or copolymerisation have a backbone that is formed by the polymerisable group P.

The mono-, oligo- and polymers of the present invention can be synthesized according to or in analogy to known methods. Some preferred methods are described in the reaction schemes below (wherein R has one of the meanings of R¹ and Ar has the meanings given above).

Materials of formula 11, wherein m is 2 and Ar is substituted arylene can occur with 3 different regioregularities with respect to the substituent at the arylene group; head-to-head; tail-to-tail and head-to-tail, like for example the compounds of formula I1a, I1b and I1c. Two general strategies to these polymers are considered, copolymerisation of two different monomers and polymerisation of a pre-formed monomer.

### Co-polymerisation

This approach is shown in Scheme 1 below and is most convenient for the synthesis of head-to-head (5) and tail-to-tail (6) polymers. The Suzuki reaction between an aryl diboronic ester or acid and an aryl dibromide is a well established route to conjugated co-polymers. The key monomer 2,7-bis(4,4,5,5-tetramethyl-1,3,2-dioxaborolan-2-yl)-fluorene (8) is synthesised from commercially available 2,7-dibromofluorene and bis(pinacolato)diboron using a palladium catalysed boronation reaction in good yield [see T. Ishiyama et al, Tetrahedron, 2001, 57, p.9813]. This is reacted with an aryldibromide such as 5,5'-dibromo-3,3'-dialkyl-2,2'-bithiophene (9) or 5,5'-dibromo-4,4"-dialkyl-2,2'-bithiophene (10) [see Bin Liu et al., Macromolecules, 2000, 33,p.8945] in the presence of a base and palladium catalyst to afford polymer 5 or 6.

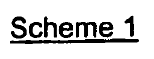

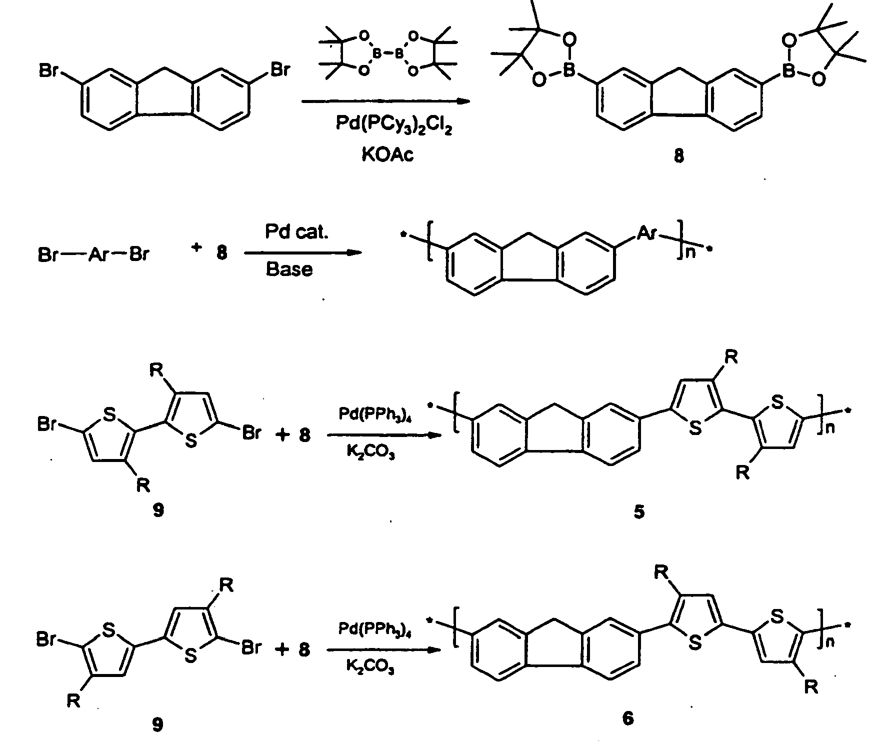

An alternate route is the Stille coupling, which involves the transition metal catalysed reaction of a diorganotin intermediate with an aryl dibromide [see B. T. Tsuie et al., J. Mater. Chem. 1999, 9, p.2189].

### Polymerisation of a pre-formed monomer

This method is shown in Scheme 2 and 3 below. The synthesis of the starting monomer can be accomplished by a variety of methods utilising cross-coupling chemistry. For monomers 11 and 12, leading to tail-to-tail and head-to-head polymers respectively, a Suzuki methodology coupling is utilised. Therefore 2,7-bis(4,4,5,5-tetramethyl-1,3,2-dioxaborolan-2-yl)-fluorene (8) is reacted with two equivalents of 2-bromo-3-alkylthiophene or 2-bromo-4-alkylthiophene to afford the monomers. Since both monomers are centro-symmetric, control of the regioregularity of the polymer is not important.

Polymerisation can occur directly via oxidative coupling using iron (III) chloride, or the monomers can be further derivatised to the dibromo derivatives and polymerised by transition metal catalysed cross-coupling reactions as shown in Scheme 3 [see T. Yamamoto, J. Organometallic Chemistry, 2002, 653, p.195].

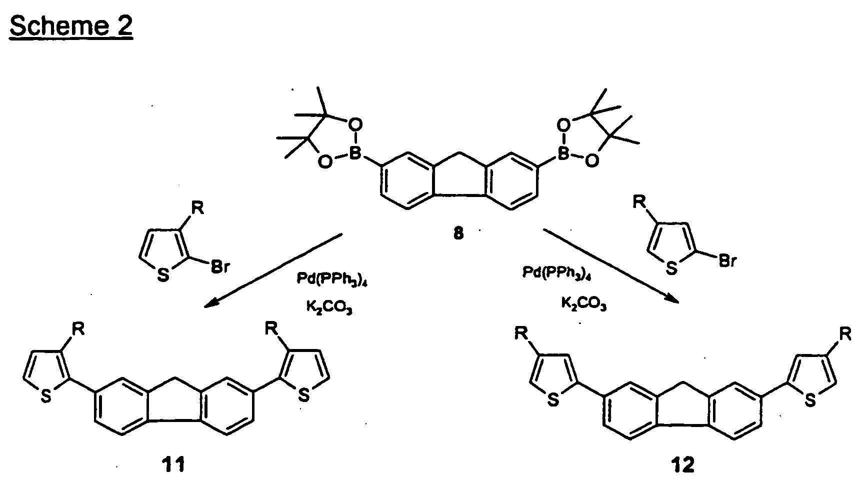

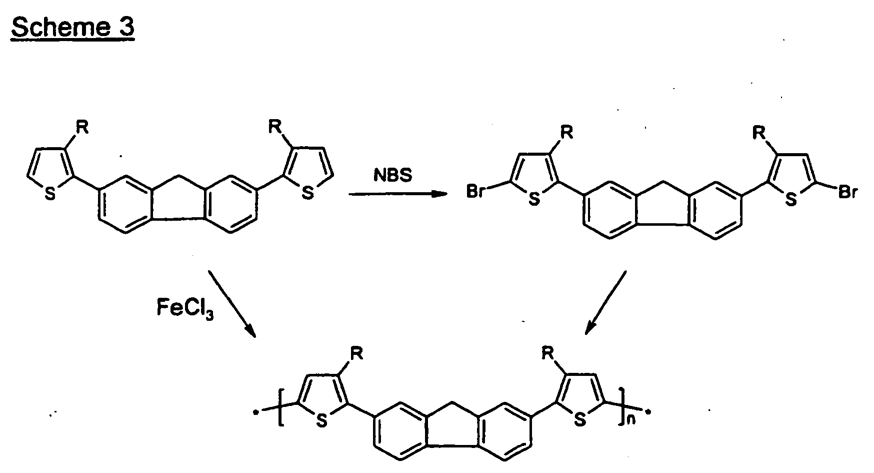

The formation of the regioregular head-to-tail polymer (7) is shown in Schemes 4 and 5 below. The monomer is synthesised in two sequential cross-coupling steps. 2-Bromo-7-iodofluorene is readily synthesised from 2-bromofluorene by iodination with iodobenzene(diacetate) and iodine. Suzuki coupling with the boronic ester of 3-alkylthiophene-2-boronate occurs at the more reactive iodo substituent [see G. Bidan et al., Chem. Mater., 1998, 10: p.1052]. A second Suzuki coupling with sodium 4-alkylthiophene-2-boronate affords the key monomer 13 (Scheme 4) [see T. Kirschbaum et al., J. Chem. Soc., Perkin Trans. 1, 2000, p.1211]. Monomer 13 is selectively monobrominated at the position ortho to the alkyl chain. Polymerisation then occurs by a method analogous to that described by McCullough and co-workers. Treatment with LDA lithiates the unbrominated thiophene ring, metathesis with zinc or magnesium bromide then forms the respective organozinc or organomagnesium reagent 'in situ'. Treatment of this with a transition metal catalyst such as Ni(dppp)Cl₂ affords polymer 7 (Scheme 5).

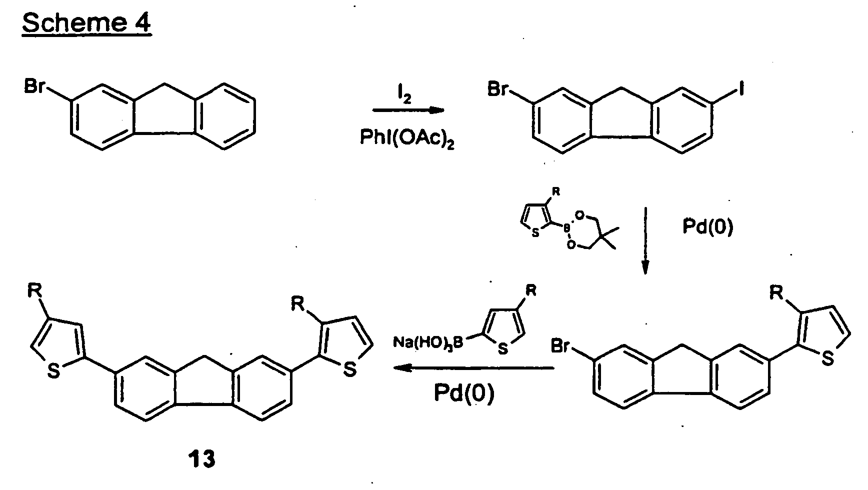

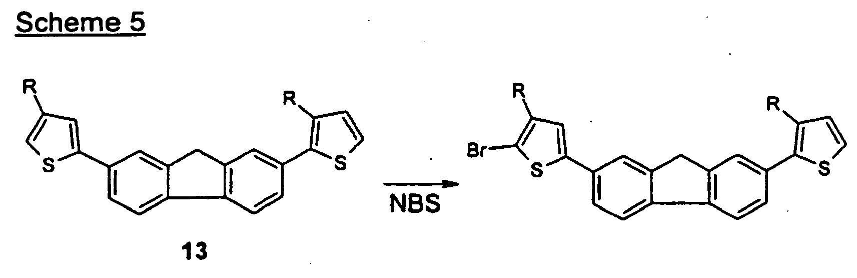

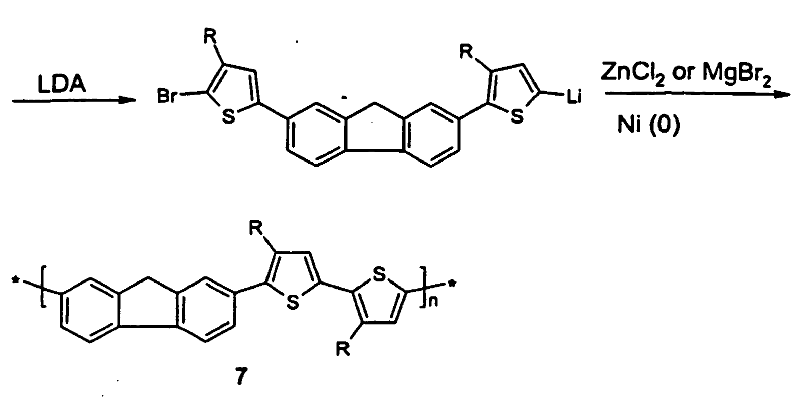

### Synthesis of monomers containing reactive end-groups

The synthesis of monomers containing reactive endgroups is outlined is scheme 6, and uitilises a Suzuki cross-coupling reaction between 2,7-bis(4,4,5,5-tetramethyl-1,3,2-dioxaborolan-2-yl)-fluorene (**8**) and an iodothiophene. The iodothiophene is synthesised in 3 steps from commercially available 2-hydroxymethylthiophene.

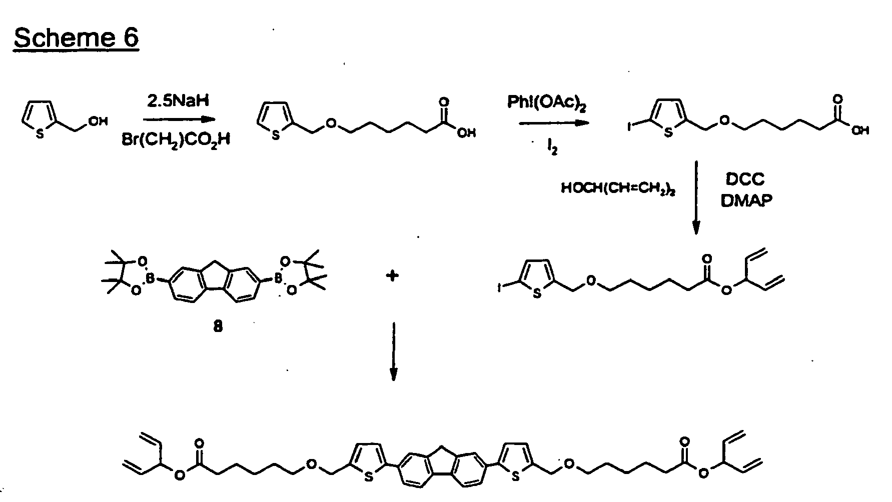

A further aspect of the invention relates to both the oxidised and reduced form of the compounds and materials according to this invention. Either loss or gain of electrons results in formation of a highly delocalised ionic form, which is of high conductivity. This can occur on exposure to common dopants. Suitable dopants and methods of doping are known to those skilled in the art, e.g., from EP 0 528 662, US 5,198,153 or WO 96/21659.

The doping process typically implies treatment of the semiconductor material with an oxidating or reducing agent in a redox reaction to form delocalised ionic centres in the material, with the corresponding counterions derived from the applied dopants. Suitable doping methods comprise for example exposure to a doping vapor in the atmospheric pressure or at a reduced pressure, electrochemical doping in a solution containing a dopant, bringing a dopant into contact with the semiconductor material to be thermally diffused, and ion-implantantion of the dopant into the semiconductor material.

When electrons are used as carriers, suitable dopants are for example halogens (e.g., I₂, CI₂, Br₂, ICI, ICI₃, IBr and IF), Lewis acids (e.g., PF₅, AsF₅, SbF₅, BF₃, BCl₃, SbCl₅, BBr₃ and SO₃), protonic acids, organic acids, or amino acids (e.g., HF, HCl, HNO₃, H₂SO₄, HClO₄, FSO₃H and ClSO₃H), transition metal compounds (e.g., FeCl₃, FeOCl, Fe(ClO₄)₃, Fe(4-CH₃C₆H₄SO₃)₃, TiCl₄, ZrCl₄, HfCl₄, NbF₅, NbCl₅, TaCl₅, MoF₅, MoCl₅, WF₅, WCl₆, UF₆ and LnCl₃ (wherein Ln is a lanthanoid), anions (e.g., Cl⁻, Br⁻ , I⁻, I₃⁻, HSO₄⁻, SO₄²-, NO₃⁻, ClO₄⁻, BF₄⁻, PF₆⁻, AsF₆⁻, SbF₆⁻, FeCl₄⁻, Fe(CN)₆³⁻, and anions of various sulfonic acids, such as aryl-SO₃⁻). When holes are used as carriers, examples of dopants are cations (e.g., H⁺, Li⁺, Na⁺, K⁺, Rb⁺ and Cs⁺), alkali metals (e.g., Li, Na, K, Rb, and Cs), alkaline-earth metals (e.g., Ca, Sr, and Ba), O₂, XeOF₄, (NO₂⁺) (SbF₆⁻), (NO₂⁺) (SbCl₆⁻), (NO₂⁺) (BF₄⁻), AgClO₄, H₂lrCl₆, La(NO₃)₃ 6H₂O, FSO₂OOSO₂F, Eu, acetylcholine, R₄N⁺, (R is an alkyl group), R₄P⁺ (R is an alkyl group), R₆As⁺ (R is an alkyl group), and R₃S⁺ (R is an alkyl group).

The conducting form of the compounds and materials of the present invention can be used as an organic "metal" in applications, for example, but not limited to, charge injection layers and ITO planarising layers in organic light emitting diode applications, films for flat panel displays and touch screens, antistatic films, printed conductive substrates, patterns or tracts in electronic applications such as printed circuit boards and condensers.

The invention further relates to a mono-, oligo- or polymer, which is oxidatively or reductively doped to form conducting ionic species. The invention further relates to a charge injection layer, planarising layer, antistatic film or conducting substrate or pattern for electronic applications or flat panel displays, comprising a mono-, oligo- or polymer as described above, or a material or polymer comprising it.

A preferred embodiment of the present invention relates to mono-, oligo- and polymers of formula I and its preferred subformulae that are mesogenic or liquid crystalline, and very preferably comprise one or more polymerisable groups. Very preferred materials of this type are monomers and oligomers of formula I and its preferred subformulae wherein n is an integer from 1 to 15 and R⁵ and/or R⁶ denote P-Sp-.

These materials are particularly useful as semiconductors or charge transport materials, as they can be aligned into uniform highly ordered orientation in their liquid crystal phase by known techniques, thus exhibiting a higher degree of order that leads to particularly high charge carrier mobility. The highly ordered liquid crystal state can be fixed by in situ polymerisation or crosslinking via the groups P to yield polymer films with high charge carrier mobility and high thermal, mechanical and chemical stability.

For example, if a device is made from a polymerisable liquid crystal material by polymerisation in situ, the liquid crystal material preferably comprises one or more mono- or oligomers of formula I and its preferred subformulae wherein one or both of R⁵ and R⁶ denote P-Sp-. If a liquid crystal polymer is preapred first, for example by polymerisation in solution, and the isolated polymer is used to make the device, the polymer is preferably made from a liquid crystal material comprising one or more mono- or oligomers of formula I and its preferred subformulae wherein one of R⁵ and R⁶ denotes P-Sp-.

It is also possible to copolymerise the polymerisable mono-, oligo- and polymers according to the present invention with other polymerisable mesogenic or liquid crystal monomers that are known from prior art, in order to induce or enhance liquid crystal phase behaviour.

Thus, another aspect of the invention relates to a polymerisable liquid crystal material comprising one or more mono-, oligo- or polymers of the present invention as described above and below comprising at least one polymerisable group, and optionally comprising one or more further polymerisable compounds, wherein at least one of the polymerisable mono-, oligo- and polymers of the present invention and/or the further polymerisable compounds is mesogenic or liquid crystalline.

Particularly preferred are liquid crystal materials having a nematic and/or smectic phase. For FET applications smectic materials are especially preferred. For OLED applications nematic or smectic materials are especially preferred.

Another aspect of the present invention relates to an anisotropic polymer film with charge transport properties obtainable from a polymerisable liquid crystal material as defined above that is aligned in its liquid crystal phase into macroscopically uniform orientation and polymerised or crosslinked to fix the oriented state.

Preferably polymerisation is carried out as in-situ polymerisation of a coated layer of the material, preferably during fabrication of the electronic or optical device comprising the inventive semiconductor material. In case of liquid crystal materials, these are preferably aligned in their liquid crystal state into homeotropic orientation prior to polymerisation, where the conjugated pi-electron systems are orthogonal to the direction of charge transport. This ensures that the intermolecular distances are minimised and hence then energy required to transport charge between molecules is minimised. The molecules are then polymerised or crosslinked to fix the uniform orientation of the liquid crystal state. Alignment and curing are carried out in the liquid crystal phase or mesophase of the material. This technique is known in the art and is generally described for example in D.J. Broer, et al., Angew. Makromol. Chem. 183, (1990), 45-66.

Alignment of the liquid crystal material can be achieved for example by treatment of the substrate onto which the material is coated, by shearing the material during or after coating, by application of a magnetic or electric field to the coated material, or by the addition of surface-active compounds to the liquid crystal material. Reviews of alignment techniques are given for example by I. Sage in "Thermotropic Liquid Crystals", edited by G. W. Gray, John Wiley & Sons, 1987, pages 75-77, and by T. Uchida and H. Seki in "Liquid Crystals - Applications and Uses Vol. 3", edited by B. Bahadur, World Scientific Publishing, Singapore 1992, pages 1-63. A review of alignment materials and techniques is given by J. Cognard, Mol. Cryst. Liq. Cryst. 78, Supplement 1 (1981), pages 1-77.

Polymerisation takes place by exposure to heat or actinic radiation. Actinic radiation means irradiation with light, like UV light, IR light or visible light, irradiation with X-rays or gamma rays or irradiation with high energy particles, such as ions or electrons. Preferably polymerisation is carried out by UV irradiation at a non-absorbing wavelength. As a source for actinic radiation for example a single UV lamp or a set of UV lamps can be used. When using a high lamp power the curing time can be reduced. Another possible source for actinic radiation is a laser, like e.g. a UV laser, an IR laser or a visible laser.

Polymerisation is preferably carried out in the presence of an initiator absorbing at the wavelength of the actinic radiation. For example, when polymerising by means of UV light, a photoinitiator can be used that decomposes under UV irradiation to produce free radicals or ions that start the polymerisation reaction. When curing polymerisable materials with acrylate or methacrylate groups, preferably a radical photoinitiator is used, when curing polymerisable materials with vinyl, epoxide and oxetane groups, preferably a cationic photoinitiator is used. It is also possible to use a polymerisation initiator that decomposes when heated to produce free radicals or ions that start the polymerisation. As a photoinitiator for radical polymerisation for example the commercially available Irgacure 651, Irgacure 184, Darocure 1173 or Darocure 4205 (all from Ciba Geigy AG) can be used, whereas in case of cationic photopolymerisation the commercially available UVI 6974 (Union Carbide) can be used.

The polymerisable material can additionally comprise one or more other suitable components such as, for example, catalysts, sensitizers, stabilizers, inhibitors, chain-transfer agents, co-reacting monomers, surface-active compounds, lubricating agents, wetting agents, dispersing agents, hydrophobing agents, adhesive agents, flow improvers, defoaming agents, deaerators, diluents, reactive diluents, auxiliaries, colourants, dyes or pigments.

Mono-, oligo- and polymers comprising one or more groups P-Sp- can also be copolymerised with polymerisable mesogenic compounds to induce or enhance liquid crystal phase behaviour. Polymerisable mesogenic compounds that are suitable as comonomers are known in prior art and disclosed for example in WO 93/22397; EP 0,261,712; DE 195,04,224; WO 95/22586 and WO 97/00600.

Another aspect of the invention relates to a liquid crystal side chain polymer (SCLCP) obtained from a polymerisable liquid crystal material as defined above by polymerisation or polymeranaloguous reaction. Particularly preferred are SCLCPs obtained from one or more monomers of formula I1 and its preferred subformulae wherein one or both of R⁷ and R⁸ are a polymerisable or reactive group, or from a polymerisable mixture comprising one or more of said monomers.

Another aspect of the invention relates to an SCLCP obtained from one or more monomers of formula I1 and its preferred subformulae wherein one or both of R⁷ and R⁸ are a polymerisable group, or from a polymerisable liquid crystal mixture as defined above, by copolymerisation or polymeranaloguous reaction together with one or more additional mesogenic or non-mesogenic comonomers.

Side chain liquid crystal polymers or copolymers (SCLCPs), in which the semiconducting component is located as a pendant group, separated from a flexible backbone by an aliphatic spacer group, offer the possibility to obtain a highly ordered lamellar like morphology. This structure consists of closely packed conjugated aromatic mesogens, in which very close (typically < 4 A) pi-pi stacking can occur. This stacking allows intermolecular charge transport to occur more easily, leading to high charge carrier mobilities. SCLCPs are advantageous for specific applications as they can be readily synthesized before processing and then e.g. be processed from solution in an organic solvent. If SCLCPs are used in solutions, they can orient spontaneously when coated onto an appropriate surface and when at their mesophase temperature, which can result in large area, highly ordered domains.

SCLCPs can be prepared from the polymerisable compounds or mixtures according to the invention by the methods described above, or by conventional polymerisation techniques which are known to those skilled in the art, including for example radicalic, anionic or cationic chain polymerisation, polyaddition or polycondensation. Polymerisation can be carried out for example as polymerisation in solution, without the need of coating and prior alignment, or polymerisation in situ.

It is also possible to form SCLCPs by grafting compounds according to the invention with a suitable reactive group, or mixtures thereof, to presynthesized isotropic or anisotropic polymer backbones in a polymeranaloguous reaction. For example, compounds with a terminal hydroxy group can be attached to polymer backbones with lateral carboxylic acid or ester groups, compounds with terminal isocyanate groups can be added to backbones with free hydroxy groups, compounds with terminal vinyl or vinyloxy groups can be added, e.g., to polysiloxane backbones with Si-H groups.

It is also possible to form SCLCPs by copolymerisation or polymeranaloguous reaction from the inventive compounds together with conventional mesogenic or non mesogenic comonomers. Suitable comonomers are known to those skilled in the art. In principle it is possible to use all conventional comonomers known in the art that carry a reactive or polymerisable group capable of undergoing the desired polymer-forming reaction, like for example a polymerisable or reactive group P as defined above. Typical mesogenic comonomers are for example those mentioned in WO 93/22397, EP 0 261 712, DE 195 04 224, WO 95/22586, WO 97/00600 and GB 2 351 734. Typical non mesogenic comonomers are for example alkyl mono- or diacrylates or alkyl mono- or dimethacrylates with alkyl groups of 1 to 20 C atoms, like methyl acrylate or methyl methacrylate, trimethylpropane trimethacrylate or pentaerythritol tetraacrylate.

The mono-, oligo- and polymers of the present invention are useful as optical, electronic and semiconductor materials, in particular as charge transport materials in field effect transistors (FETs), e.g., as components of integrated circuitry, ID tags or TFT applications. Alternatively, they may be used in organic light emitting diodes (OLEDs) in electroluminescent display applications or as backlight of, e.g., liquid crystal displays, as photovoltaics or sensor materials, for electrophotographic recording, and for other semiconductor applications.

Especially the oligomers and polymers according to the invention show advantageous solubility properties which allow production processes using solutions of these compounds. Thus films, including layers and coatings, may be generated by low cost production techniques, e.g., spin coating. Suitable solvents or solvent mixtures comprise alkanes and/ or aromatics, especially their fluorinated derivatives.

The materials of the present invention are useful as optical, electronic and semiconductor materials, in particular as charge transport materials in field effect transistors (FETs), as photovoltaics or sensor materials, for electrophotographic recording, and for other semiconductor applications. Such FETs, where an organic semiconductive material is arranged as a film between a gate-dielectric and a drain and a source electrode, are generally known, e.g., from US 5,892,244, WO 00/79617, US 5,998,804, and from the references cited in the background and prior art chapter and listed below. Due to the advantages, like low cost production using the solubility properties of the compounds according to the invention and thus the processibility of large surfaces, preferred applications of these FETs are such as integrated circuitry, TFT-displays and security applications.

In security applications, field effect transistors and other devices with semiconductive materials, like transistors or diodes, may be used for ID tags or security markings to authenticate and prevent counterfeiting of documents of value like banknotes, credit cards or ID cards, national ID documents, licenses or any product with money value, like stamps, tickets, shares, cheques etc..

Alternatively, the mono-, oligo- and polymers according to the invention may be used in organic light emitting devices or diodes (OLEDs), e.g., in display applications or as backlight of e.g. liquid crystal displays. Common OLEDs are realized using multilayer structures. An emission layer is generally sandwiched between one or more electron-transport and/ or hole-transport layers. By applying an electric voltage electrons and holes as charge carriers move towards the emission layer where their recombination leads to the excitation and hence luminescence of the lumophor units contained in the emission layer. The inventive compounds, materials and films may be employed in one or more of the charge transport layers and/or in the emission layer, corresponding to their electrical and/ or optical properties. Furthermore their use within the emission layer is especially advantageous, if the compounds, materials and films according to the invention show electroluminescent properties themselves or comprise electroluminescent groups or compounds. The selection, characterization as well as the processing of suitable monomeric, oligomeric and polymeric compounds or materials for the use in OLEDs is generally known by a person skilled in the art, see, e.g., Meerholz, Synthetic Materials, 111-112, 2000, 31-34, Alcala, J. Appl. Phys., 88, 2000, 7124-7128 and the literature cited therein.

According to another use, the inventive compounds, materials or films, especially those which show photoluminescent properties, may be employed as materials of light sources, e.g., of display devices such as described in EP 0 889 350 A1 or by C. Weder et al., Science, 279, 1998, 835-837.

According to another use, the inventive compounds, materials or films can be used alone or together with other materials in or as alignment layers in LCD or OLED devices, as described for example in US 2003/0021913. The use of charge transport compounds according to the present invention can increase the electrical conductivity of the alignment layer. When used in an LCD, this increased electrical conductivity can reduce adverse residual dc effects in the switchable LCD cell and suppress image sticking or, for example in ferroelectric LCDs, reduce the residual charge produced by the switching of the spontaneous polarisation charge of the ferroelectric LCs. When used in an OLED device comprising a light emitting material provided onto the alignment layer, this increased electrical conductivity can enhance the electroluminescence of the light emitting material. The compounds or materials according to the present invention having mesogenic or liquid crystalline properties can form oriented anisotropic films as described above, which are especially useful as alignment layers to induce or enhance alignment in a liquid crystal medium provided onto said anisotropic film. The materials according to the present invention may also be combined with photoisomerisable compounds and/or chromophores for use in or as photoalignment layers, as described in US 2003/0021913.

The invention is further explained by the following examples.

### Example 1

Poly(3,3'-dihexyl-2,2'-bithiophene-*alt*-fluorene) of formula Ia, wherein R¹ is n-hexyl, was prepared according to Scheme 1.

**2,7-Bis(4,4,5,5-tetramethyl-1,3,2-dioxaborolan-2-yl)-fluorene (8)** 2,7-Dibromofluorene (5.74 g, 17.73 mmol), bis(pinacolato)diboron (11.26 g, 44.33 mmol), potassium acetate (5.22 g, 53.20 mmol) and dichlorobis (tricyclohexylphosphine)palladium(II) (0.78 g, 0.61 mmol) were charged in a 3-necked flask. Anhydrous 1,4-dioxane (150 mL) was added and the reaction mixture was stirred at 100°C for 24 h. The reaction mixture was quenched with water (100 mL) and extracted into chloroform (2 x 200 mL). The combined extracts were washed with water (100 mL), dried over sodium sulfate and concentrated *in vacuo.* Purifcation by column chromatography (eluent: dichloromethane) yielded the product as a white solid (5.67 g, 13.56 mmol, 76%). NMR shows the expected signals. M⁺ = 418.

**3,3'-Dihexyl-2,2'-bithiophene** was prepared according to the method of P. Bäuerle, F. Pfau, H. Schlupp, F. Würthner, K. U. Gaudl, M. B. Caro, P. Fischer, J. Chem. Soc., Perkin Trans. 2,1993, 489.

**5,5'-Dibromo-3,3'-Dihexyl-2,2'-bithiophene (9) (R = C₆H₁₃)** was prepared according to the method of B. Liu, W. L. Yu, Y. H. Lai, W. Huang, Macromolecules, 2000, 33, 8945.

**4,4'-Didodecyl-2,2'-bithiophene**
2.5M BuLi in hexanes (18 mL, 45.00 mmol) and anhydrous TMEDA (6.8 mL, 45.06 mmol) were added dropwise to a solution of 3-dodecylthiophene (10.00 g, 39.61 mmol) in anhydrous THF (40 mL) at RT under nitrogen. The reaction mixture was stirred at reflux for 1 h and then cooled down to -78°C. Copper(II) chloride (6.39 g, 47.53 mmol) was added in one portion and the reaction mixture was allowed to warm to RT stirring for 18 h. The reaction mixture was then stirred at reflux for 6 h. The reaction mixture was acidified with dilute hydrochloric acid and extracted into diethyl ether (2 x 200 mL). The combined extracts were washed with water (100 mL), dried over anhydrous sodium sulfate and concentrated *in vacuo.* Column chromatography (eluent: petroleum ether 40-60) followed by recrystallisation from diethyl ether at -78°C yielded a yellow solid (3.78 g, 7.52 mmol, 38%): δ_{H}(CDCl₃, 300 MHz) 6.97 (2H, s), 6.76 (2H, s), 2.56 (4H, t, ³J_{HH} = 8.0 Hz), 1.61 (4H, m), 1.20-1.40 (36H, br), 0.88 (6H, t, ³J_{HH} = 7.0 Hz); δ_{c}(CDCl₃, 75 MHz) 144.0, 137.4, 124.8, 118.7, 32.0, 30.6, 30.4, 29.7, 29.6, 29.5, 29.4, 29.3, 22.7, 14.2.

**5,5'-Dibromo-4,4'-didodecyl-2,2'-bithiophene (10) (R = C₁₂H₂₅)** NBS (0.53 g, 2.98 mmol) was added in portions to 4,4'-didodecyl-2,2'-bithiophene (0.75 g, 1.49 mmol) in chloroform (5 mL) and glacial acetic acid (5 mL) under nitrogen. The reaction mixture was stirred overnight before being poured onto water and extracted into DCM (2 x 250 mL). The combined extracts were washed with water (2 x 100 mL), dried over anhydrous sodium sulfate, and concentrated in *vacuo.* Column chromatography (eluent: petroleum ether 40-60) yielded the product as a yellow solid (0.99 g, 1.49 mmol, 100%): δ_{H}(CDCl₃, 300 MHz) 6.77 (2H, s), 2.51 (4H, t, ³J_{HH} = 8.0 Hz), 1.57 (4H, m), 1.20-1.40 (36H, br), 0.88 (6H, t, ³J_{HH} = 7.0 Hz); δ_{c}(CDCl₃, 75 MHz) 142.8, 136.0, 124.3, 107.0, 31.8, 29.5, 29.4, 29.2, 29.0, 22.6, 14.0.

**Poly(3,3'-dihexyl-2,2'-bithiophene-alt-fluorene) (5) (R = C₆H₁₃)** A 3-necked flask was charged with 2,7-bis(4,4,5,5-tetramethyl-1,3,2-dioxaborolan-2-yl)-fluorene (8) (1.28 g, 3.07 mmol), 5,5'-dibromo-3,3'-dihexyl-2,2'-bithiophene (1.51 g, 3.07 mmol), tetrakis(triphenylphosphine)palladium(0) (34 mg, 0.03 mmol), Aliquat 336 (0.50 g) and toluene (30 mL) under nitrogen. A 2M aqueous solution of sodium carbonate (5 mL) was added and the reaction mixture was stirred at reflux for 22 h. The reaction mixture was precipitated into methanol (500 mL). The polymer was collected and washed with water followed by methanol. After drying, the polymer was dissolved in hot chloroform and reprecipitated from methanol (500 mL). The polymer was collected and washed with refluxing methanol (via Soxhlet extraction) for 18 h followed by refluxing iso-hexane (via Soxhlet extraction) for 6 h. The polymer was then redissolved in hot chloroform and reprecipitated from methanol (400 mL). The polymer was collected and dried under vacuum to yield the polymer as a lime green solid (1.39 g, 2.79 mmol, 91 %): m.p. 122°C (Tg); 199 (I).δₕ(CDCl₃, 500 MHz) 7.80 (2H, s), 7.77 (2H, d, ³J_{HH} = 8.0 Hz), 7.66 (2H, d, ³J_{HH} = 8.0 Hz), 7.27 (2H, s), 3.99 (2H, s), 2.62 (4H, br), 1.65 (4H, br), 1.30-1.35 (12H, br), 0.88 (6H, br). M_{w} = 52,000, Mₙ = 26,000; abs λₘₐₓ (CDCl₃) 406 nm.

### Example 2

Poly(4,4'-didodecyl-2,2'-bithiophene-alt-fluorene) of formula Ib, wherein R¹ is n-dodecyl, was prepared according to Scheme 1.

**Poly(4,4'-didodecyl-2,2'-bithiophene-*alt*-fluorene) (6) (R = C₁₂H₂₅)** A 3-necked flask was charged with 2,7-bis(4,4,5,5-tetramethyl-1,3,2-dioxaborolan-2-yl)-fluorene (8) (0.60 g, 1.44 mmol), 5,5'-dibromo-4,4'-didodecyl-2,2'-bithiophene (0.95 g, 1.44 mmol), tetrakis(triphenylphosphine)palladium(0) (34 mg, 0.03 mmol), Aliquat 336 (0.25 g) and toluene (20 mL) under nitrogen. A 2M aqueous solution of sodium carbonate (2.5 mL) was added and the reaction mixture was stirred at reflux for 48 h. The reaction mixture was precipitated into methanol (400 mL). The polymer was collected and washed with water followed by methanol. After drying, the polymer was washed with methanol *(via* Soxhlet extraction) for 16 h followed by iso-hexane *(via* Soxhlet extraction) for 6 h. The polymer was then redissolved in hot chloroform and reprecipitated from methanol (400 mL). The polymer was collected and dried under vacuum to yield the polymer as a green solid (0.75 g, 1.12 mmol, 78%): m.p. 130° (Tg), 160°C (N), 360°C (dec.) δ_{H}(CDCl₃, 300 MHz) 7.84 (2H, d, ³J_{HH} = 8.0 Hz), 7.66 (2H, s), 7.51 (2H, d, ³J_{HH} = 8.0 Hz), 7.11 (2H, s), 4.02 (2H, s), 2.71 (4H, br), 1.67 (4H, br), 0.95-1.40 (36H, br), 0.87 (6H, br). M_{w} = 137,000, Mₙ = 32,000 (bimodal); abs λₘₐₓ (CDCl₃) 405 nm.

### Example 3

Poly(9-((bishexyl-methylene)fluorene-*alt*-fluorene)) of formula Ig, wherein R¹ is n-hexyl, was prepared according to Scheme 1.

**Poly(9-((bishexyl-methylene)fluorene-alt-fluorene)**
A 3-necked flask was charged with 2,7-bis(4,4,5,5-tetramethyl-1,3,2-dioxaborolan-2-yl)-fluorene (8) (0.622 g, 1.49 mmol), 2,7-dibromo-9-(bis-hexyl-methylene)fluorene (0.75 g, 1.49 mmol), tetrakis(triphenylphosphine)palladium(0) (17 mg, 0.015 mmol), Aliquat 336 (0.25 g) and toluene (20 mL) under nitrogen. A 2M aqueous solution of sodium carbonate (2.5 mL) was added and the reaction mixture was stirred at reflux for 22 h. Bromobenzene (0.12g, 0.75 mmol) was added and the solution was stirred for 1 h at reflux, then bezene boronic acid was added and the solution stirred for a further 1 h at reflux. The reaction mixture was cooled, precipitated into methanol (400 mL). The polymer was collected and washed with water followed by methanol. After drying, the polymer was washed with methanol (via Soxhlet extraction) for 24 h followed by iso-hexane *(via* Soxhlet extraction) for 24 h to afford the polymer as a green solid (0.73g) of poor solubility m.p.> 300°C.

### Example 4

Monomer (4) was prepared according to Scheme 6 as follows.

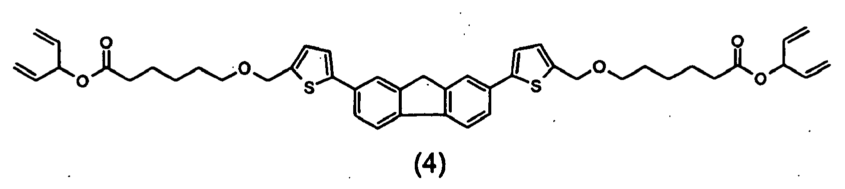

Tetrakis(triphenylphosphine)palladium(0) (0.05 g) was added to a solution of vinylallyl 6-[5-iodo-(2-thienyl)] methoxyhexanoate (0.84g, 2.0 mmol) in dry DME (70 ml) with stirring under nitrogen. After 20 min, 5,5'-di(4,4,5,5-tetramethyl[1,3,2]dioxoborolan-2-yl)-2,2'-bithiophene (0.20 g, 0.48 mmol) was added, followed by the addition of cesium fluoride (0.46 g, 3.0 mmol). The reaction mixture was heated at reflux overnight under nitrogen. After cooling, water (50 ml) was added, followed by extraction with ethyl acetate (3 x 50 ml). The extracts were washed with water (50 ml) and brine (50 ml), dried (Na₂SO₄) and evaporated under reduced pressure. Purification by column chromatography on silica, eluting with petroleum ether/ethyl acetate (from 9:1 to 5:1), gave a yellow solid, which was recystallised from ethyl acetate twice to afford 2 as pale yellow crystals (0.16 g. 44 %). ¹H NMR (300 MHz, CDCl₃): δ (ppm) 7.65-7.78 (m, 6H, Ar-H), 7.23 (d, J = 3.6 Hz, 2H, Ar-H), 6.98 (d, J = 3.6 Hz, 2H, Ar-H), 5.87 (m, 4H, =CH), 5.83 (m, 2H, OCH), 5.28 (m, 8H, =CH₂), 4.68 (s, 4H, OCH₂), 3.98 (s, 2H, CH₂), 3.54 (t, *J* = 8.3 Hz), 2.38 (t, *J* = 7.4 Hz, 4H, CO-CH₂), 1.67 (m, 8H, CH₂), 1.47 (m, 4H, CH₂); ¹³C NMR (75 MHz, CDCl₃): δ (ppm) 173.0 (2 x C=O), 145.5 (2 x quat.), 144.5 (2 x quat.), 141.13 (2 x quat.), 141.10 (2 x quat.), 135.5 (4 x CH), 133.5 (2 x quat.), 127.7 (2 x CH), 125.1 (2 x CH), 122.8 (2 x CH), 122.7 (2 x CH), 120.5 (2 x CH), 117.8 (4 x CH₂), 75.2 (2 x CH₂), 70.3 (2 x CH₂), 68.0 (2 x CH₂), 37.3 (2 x quat.), 34.9 (2 x CH₂), 29.8 (2 x CH₂), 26.1 (2 x CH₂), 25.2 (2 x CH₂); MS (m/e): 768 (M⁺ + OH, 100 %), 553 (66), 242 (74), 129 (10).

The following phase transitions and liquid crystal phase behaviour were determined by optical microscopy and DSC: K-30-Sₓ 146-I were Sₓ is an undetermined highly order smectic phase.

## Claims

1. Mono-, oligo- or polymers selected of formula 11 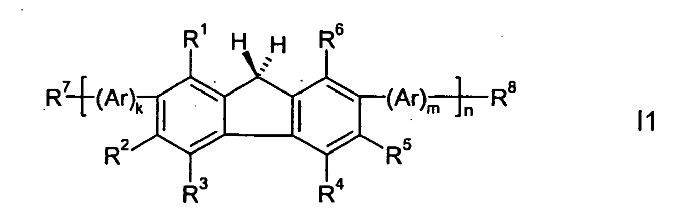 wherein
R¹ to R⁶ are independently of each other H, halogen or straight chain, branched or cyclic alkyl with 1 to 20 C-atoms, which may be unsubstituted, mono- or polysubstituted by F, Cl, Br, I and/or CN, it being also possible for one or more non-adjacent CH₂ groups to be replaced, in each case independently from one another, by -O-, -S-, -NH-, -NR⁰-,-SiR⁰R⁰⁰-, -CO-, -COO-, -OCO-, -OCO-O-, -S-CO-,-CO-S-, -CH=CH- or -C≡C- in such a manner that O and/or S atoms are not linked directly to one another, optionally substituted aryl or heteroaryl, or P-Sp-,
P is a polymerisable or reactive group,
Sp is a spacer group or a single bond,
R⁰ and R⁰⁰ are independently of each other H or alkyl with 1 to 12 C-atoms,
Ar is is an arylene or heteroarylene group that is optionally substituted with one or more groups R¹, and
k and m are independently of each other 0, 1, 2, 3 or 4, with k+m > 1, or k is 0 or 1 and m is 1 or 2,
R⁷ and R⁸ are independently of each other H, halogen, Sn(R⁰)₃ or straight chain, branched or cyclic alkyl with 1 to 20 C-atoms, which may be unsubstituted, mono- or polysubstituted by F, Cl, Br, I, CN and/or OH, it being also possible for one or more non-adjacent CH₂ groups to be replaced, in each case independently from one another, by -O-, -S-, -NH-,-NR⁰-, -SiR⁰R⁰⁰-, -CO-, -COO-, -OCO-, -OCO-O-,-S-CO-, -CO-S-, -CH=CH- or -C≡C- in such a manner that O and/or S atoms are not linked directly to one another, optionally substituted aryl or heteroaryl, or P-Sp,
n is an integer from 1 to 10000,
wherein the recurring units may be identical or different,
wherein
n is from 2 to 5000, or
n is an integer from 1 to 15 and one or both of R⁷ and R⁸ denote P-Sp-, or
R⁷ and R⁸ are selected from halogen, Sn(R⁰)₃, CH₂Cl, COH, CH=CH₂, SiR⁰R⁰⁰.

2. Oligo- or polymers according to claim 1 with a degree of polymerisation from 10 to 5000.

3. Oligo- or polymers according to claim 1 or 2 having identical recurring units.

4. Mono-, oligo- or polymers according to at least one of claims 1 to 3, wherein R¹ to R⁶ are H.

5. Mono-, oligo- or polymers according to at least one of claims 1 to 4, wherein R¹ to R⁶ are H selected from C₁-C₂₀-alkyl that is optionally substituted with one or more fluorine atoms, C₁-C₂₀-alkenyl, C₁-C₂₀-alkynyl, C₁-C₂₀-alkoxy, C₁-C₂₀-thioether, C₁-C₂₀-silyl, C₁-C₂₀-ester, C₁-C₂₀-amino, C₁-C₂₀-fluoroalkyl, (CH₂CH₂O)ₘCᵢH₂ᵢ₊₁, with m being from 1 to 6, i being 1, 2 or 3 and optionally substituted aryl or heteroaryl.

6. Mono-, oligo- or polymers according to at least one of claims 1 to 5, wherein Ar is selected from phenyl, fluorinated phenyl, pyridine, pyrimidine, biphenyl, naphthalene, thiophene, fluorinated thiophene, benzo[1,2-b:4,5-b']dithiophene, cyclopenta[2,1-b:3,4-b']dithiophene, 9-alkylidenefluorene, 9-H,H-fluorene, thiazole and oxazole, all of which are unsubstituted, mono- or polysubstituted with R¹ as defined in claim 1 or with L, wherein L is F, Cl, Br, or an alkyl, alkoxy, alkylcarbonyl or alkoxycarbonyl group with 1 to 12 C atoms, wherein one or more H atoms are optionally replaced by F or Cl.

7. Mono-, oligo- or polymers according to claim 6, wherein Ar is thiophene-2,5-diyl that is optionally substituted in 3- or 4-position by R¹.

8. Mono-, oligo- or polymers according to at least one of claims 1 to 7, selected from the following formulae 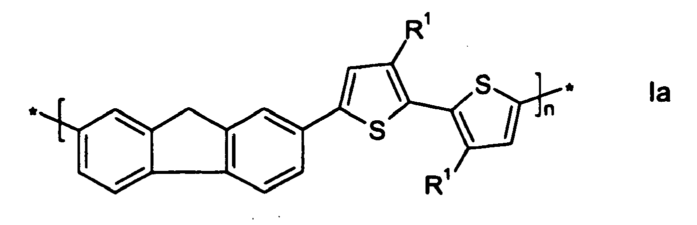 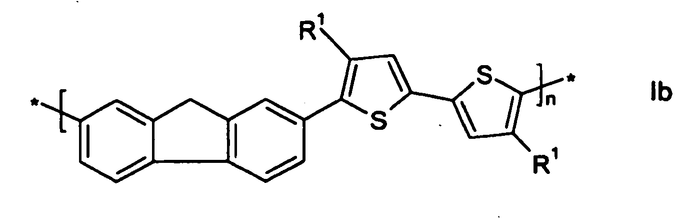 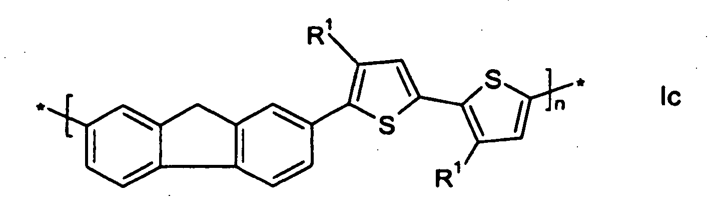 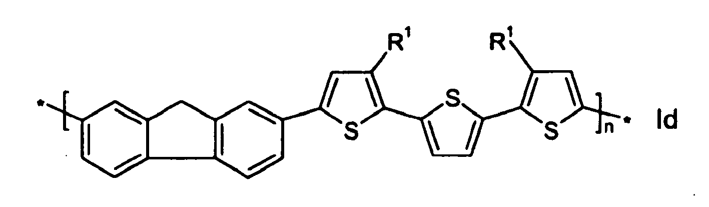 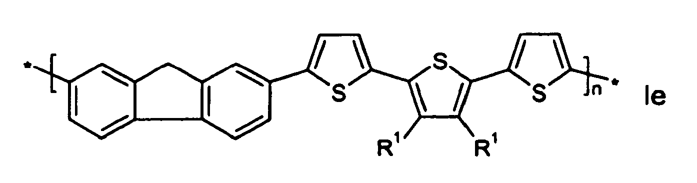 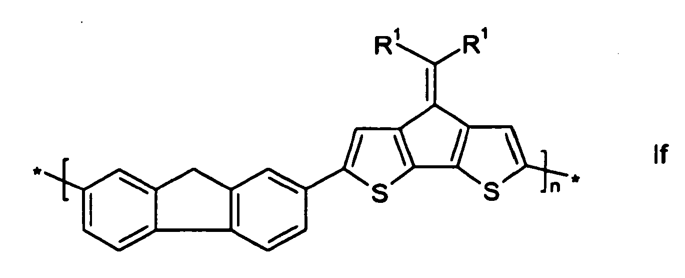 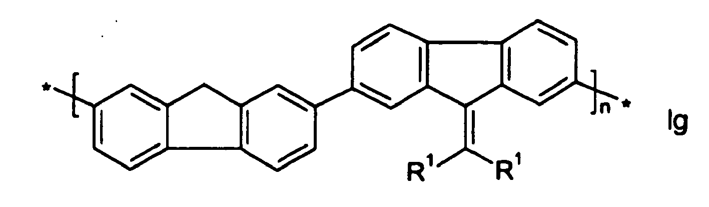 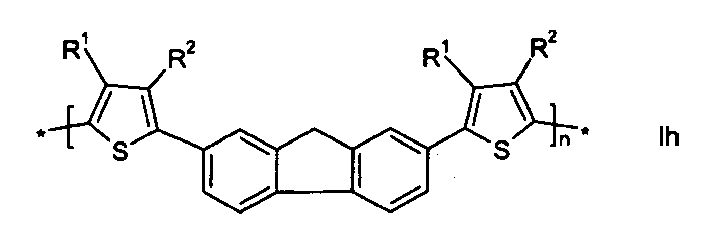 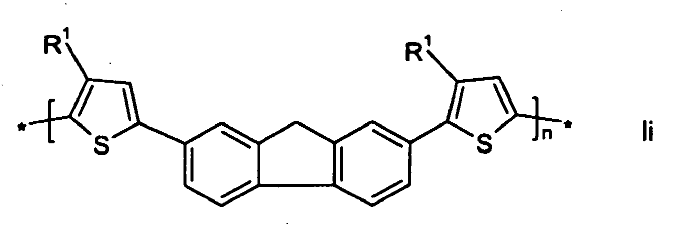 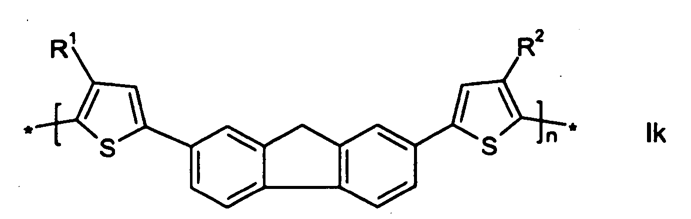 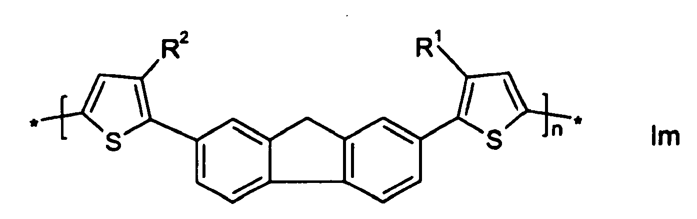 wherein n, R¹ and R² have the meanings given in claim 1.

9. Monomers according to at least one of claims 1 to 8, selected from the following formulae 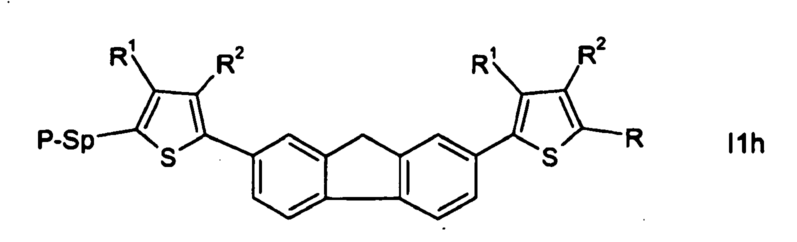 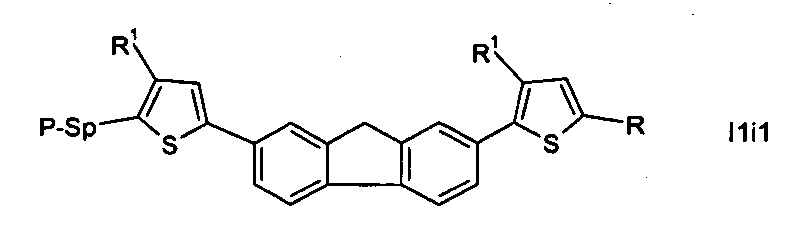 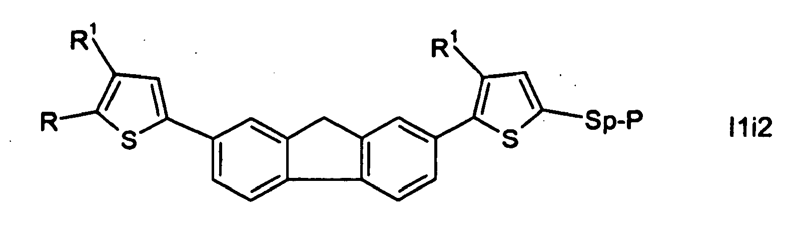 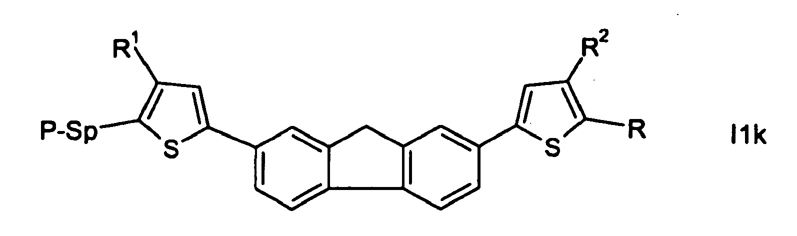 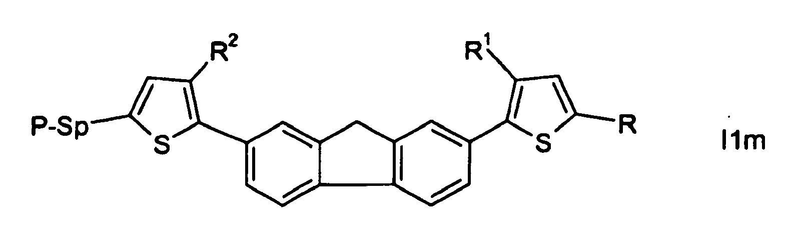 wherein P, Sp, R¹ and R² are as defined in claim 1 and R has one of the meanings of R⁷ in claim 1.

10. Monomers according to claim 9, wherein R is P-Sp.

11. Mono-, oligo- or polymers according to at least one of claims 1 to 10, wherein R⁷, R⁸ and R are selected from H, halogen, Sn(R⁰)₃ , CH₂Cl, COH, CH=CH_{2,} SiR⁰R⁰⁰ and optionally substituted aryl or heteroaryl.

12. Mono-, oligo- or polymers according to at least one of claims 1 to 11, wherein P is selected from CH₂=CW¹-COO-, 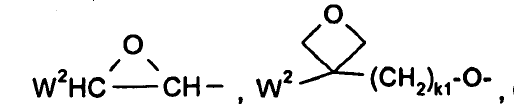 CH₂=CW²-(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN-, and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene that is optionally substituted by one or more groups R¹ as defined in claim 1, and k₁ and k₂ being independently of each other 0 or 1.

13. Polymerisable liquid crystal material comprising one or more mono-, oligo- or polymers according to at least one of claims 1 to 12 comprising at least one polymerisable group, and optionally comprising one or more further polymerisable compounds, wherein at least one of the polymerisable mono-, oligo- and polymers of claims 1 to 12 and/or the further polymerisable compounds is mesogenic or liquid crystalline.

14. Anisotropic polymer film with charge transport properties obtainable from a polymerisable liquid crystal material according to claim 13 that is aligned in its liquid crystal phase into macroscopically uniform orientation and polymerised or crosslinked to fix the oriented state.

15. Side chain liquid crystal polymer obtained by polymerisation of one or more mono- or oligomers or a polymerisable material according to at least one of claims 1 to 13 or by grafting one or more mono- or oligomers or a polymerisable material according to at least one of claims 1 to 13 to a polymer backbone in a polymeranaloguous reaction, optionally with one or more additional mesogenic or non-mesogenic comonomers.

16. Use of the mono-, oligo- and polymers, polymerisable materials and polymers according to at least one of claims 1 to 17 as semiconductors or charge transport materials in optical, electrooptical or electronic devices, like field effect transistors (FET) for example as components of integrated circuitry, of thin film transistors (TFT) for flat panel display applications, or of radio frequency identification (RFID) tags, or semiconducting components for organic light emitting diode (OLED) applications including both the charge transport and electroluminescent layers in electroluminescent displays or backlights of liquid crystal displays (LCD).

17. Use of the mono-, oligo- and polymers, polymerisable materials and polymers according to at least one of claims 1 to 15 as electroluminescent material, in photovoltaic or sensor devices, as electrode material in batteries, as photoconductor, for electrophotographic applications like electrophotographic recording and as alignment layer in LCD or OLED devices.

18. Optical, electrooptical or electronic device, FET, integrated circuit (IC), TFT, OLED or alignment layer comprising a material, component or device according to at least one of claims 1 to 15.

19. TFT or TFT array for flat panel displays, radio frequency identification (RFID) tag, electroluminescent display or backlight, comprising a material, component or device according to at least one of claims 1 to 15 or a FET, IC, TFT or OLED according to claim 18.

20. Security marking or device comprising a FET or an RFID tag according to claim 18 or 19.

21. Mono-, oligo- and polymer, material or polymer as defined in at least one of claims 1 to 15, which is oxidatively or reductively doped to form conducting ionic species.

22. Charge injection layer, planarising layer, antistatic film or conducting substrate or pattern for electronic applications or flat panel displays, comprising a mono-, oligo- or polymer, material or polymer according to claim 21.

## Patentansprüche

1. Mono-, Oligo- oder Polymere ausgewählt aus der Formel I1 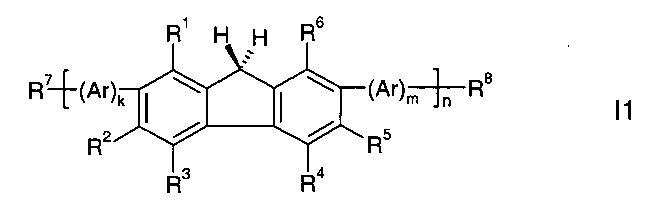 worin
R¹ bis R⁶ unabhängig voneinander H, Halogen oder geradkettiges, verzweigtes oder cyclisches Alkyl mit 1 bis 20 C-Atomen, welches unsubstituiert oder ein- oder mehrfach durch F, Cl, Br, I und/oder CN substituiert sein kann, wobei auch eine oder mehrere nicht benachbarte CH₂-Gruppen jeweils unabhängig voneinander so durch -O-, -S-, -NH-, -NR⁰-, -SiR⁰R⁰⁰-, -CO-, -COO-, -OCO-, -OCO-O-, -S-CO-, -CO-S-, -CH=CH- oder -C≡C- ersetzt sein können, dass O- und/oder S-Atome nicht direkt miteinander verknüpft sind, gegebenenfalls substituiertes Aryl oder Heteroaryl, oder P-Sp- bedeuten,
P eine polymerisierbare oder reaktive Gruppe bedeutet,
Sp eine Spacergruppe oder eine Einfachbindung bedeutet,
R⁰ und R⁰⁰ unabhängig voneinander H oder Alkyl mit 1 bis 12 C-Atomen bedeuten,
Ar eine Arylen- oder Heteroarylengruppe bedeutet, welche gegebenenfalls mit einer oder mehreren Gruppen R¹ substituiert ist, und
k und m unabhängig voneinander 0, 1, 2, 3 oder 4 bedeuten, wobei k+m > 1, oder k 0 oder 1 bedeutet und m 1 oder 2 bedeutet,
R⁷ und R⁸ unabhängig voneinander H, Halogen, Sn(R⁰)₃ oder geradkettiges, verzweigtes oder cyclisches Alkyl mit 1 bis 20 C-Atomen; welches unsubstituiert oder ein- oder mehrfach durch F, Cl, Br, I, CN und/oder OH substituiert sein kann, wobei auch eine oder mehrere nicht benachbarte CH₂-Gruppen jeweils unabhängig voneinander so durch -O-, -S-, -NH-, -NR⁰-, -SiR⁰R⁰⁰-, -CO-, -COO-, -OCO-, -OCO-O-, -S-CO-, -CO-S-, -CH=CH- oder -C≡C- ersetzt sein können, dass O- und/oder S-Atome nicht direkt miteinander verknüpft sind, gegebenenfalls substituiertes Aryl oder Heteroaryl, oder P-Sp bedeuten,
n eine ganze Zahl von 1 bis 10000 bedeutet,
worin die wiederkehrenden Einheiten gleich oder verschieden sein können,
worin
n 2 bis 5000 bedeutet, oder
n eine ganze Zahl von 1 bis 15 bedeutet und eines oder beide von R⁷ und R⁸ für P-Sp- stehen, oder
R⁷ und R⁸ ausgewählt sind aus Halogen, Sn(R⁰)₃, CH₂Cl, COH, CH=CH_{2,} SiR⁰R⁰⁰_{.}

2. Oligo- oder Polymere nach Anspruch 1 mit einem Polymerisationsgrad von 10 bis 5000.

3. Oligo- oder Polymere nach Anspruch 1 oder 2 mit gleichen wiederkehrenden Einheiten.

4. Mono-, Oligo- oder Polymere nach mindestens einem der Ansprüche 1 bis 3, worin R¹ bis R⁶ H bedeuten.

5. Mono-, Oligo- oder Polymere nach mindestens einem der Ansprüche 1 bis 4, worin R¹ bis R⁶ ausgewählt sind aus C₁-C₂₀-Alkyl, welches gegebenenfalls mit einem oder mehreren Fluoratomen substituiert ist, C₁-C₂₀-Alkenyl, C₁-C₂₀-Alkinyl, C₁-C₂₀-Alkoxy, C₁-C₂₀-Thioether, C₁-C₂₀-Silyl, C₁-C₂₀-Ester, C₁-C₂₀-Amino, C₁-C₂₀-Fluoralkyl, (CH₂CH₂O)ₘCⱼH₂ᵢ₊₁, wobei m 1 bis 6 bedeutet, i 1, 2 oder 3 bedeutet, und gegebenenfalls substituiertem Aryl oder Heteroaryl.

6. Mono-, Oligo- oder Polymere nach mindestens einem der Ansprüche 1 bis 5, worin Ar ausgewählt ist aus Phenyl, fluoriertem Phenyl, Pyridin, Pyrimidin, Biphenyl, Naphthalin, Thiophen, fluoriertem Thiophen, Benzo[1,2-b:4,5-b']dithiophen, Cyclopenta[2,1-b:3,4-b']dithiophen, 9-Alkylidenfluoren, 9-H,H-Fluoren, Thiazol und Oxazol, die alle unsubstituiert oder ein- oder mehrfach mit R¹ wie in Anspruch 1 definiert oder mit L substituiert sind, worin L F, Cl, Br oder eine Alkyl-, Alkoxy-, Alkylcarbonyl- oder Alkoxycarbonylgruppe mit 1 bis 12 C-Atomen bedeutet, worin ein oder mehrere H-Atome gegebenenfalls durch F oder Cl ersetzt sind.

7. Mono-, Oligo- oder Polymere nach Anspruch 6, worin Ar Thiophen-2,5-diyl bedeutet, welches gegebenenfalls in 3- oder 4-Position durch R¹ substituiert ist.

8. Mono-, Oligo- oder Polymere nach mindestens einem der Ansprüche 1 bis 7, ausgewählt aus den folgenden Formeln 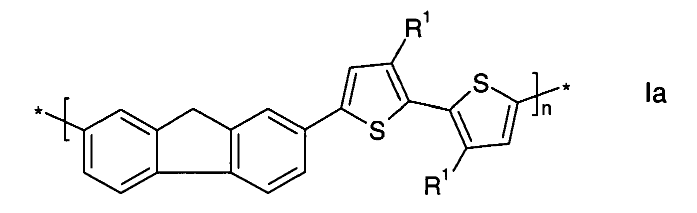 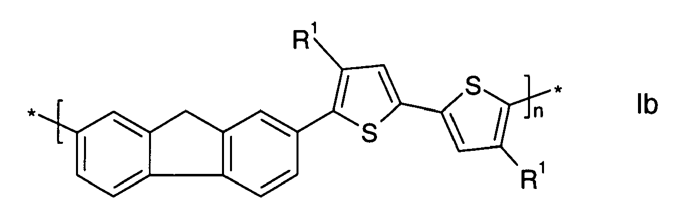 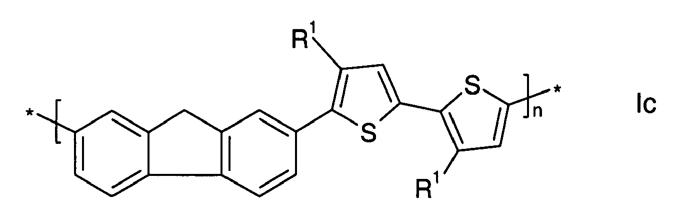 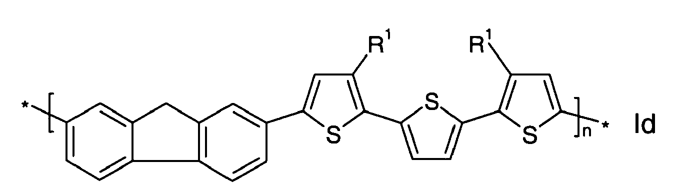 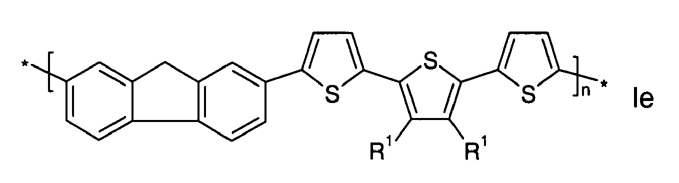 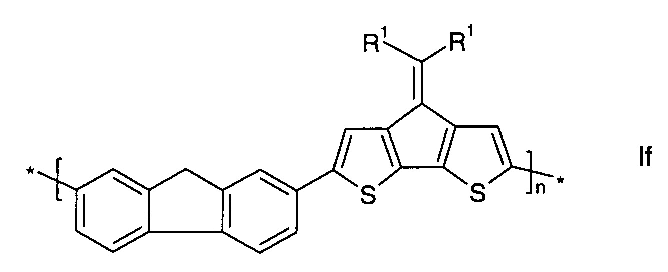 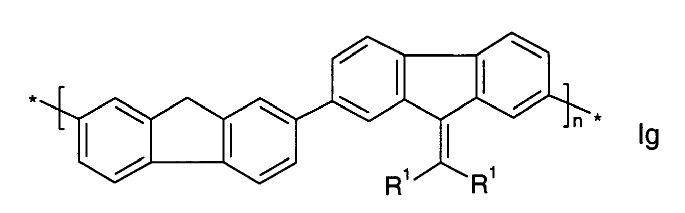 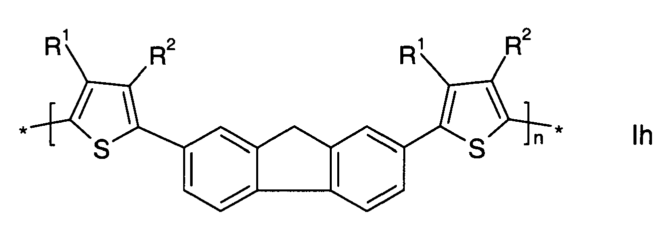 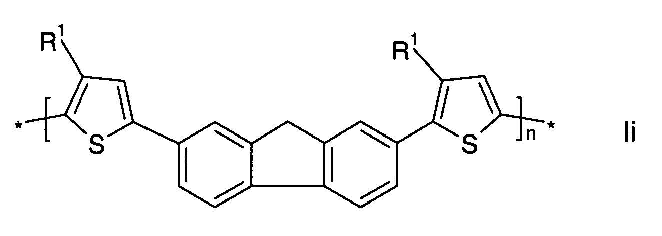 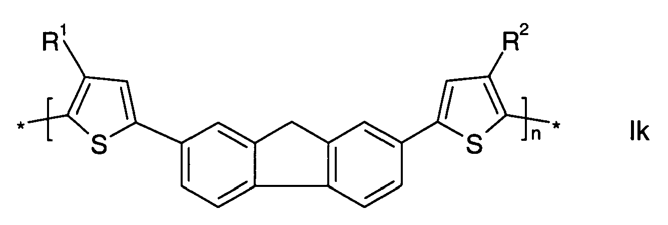 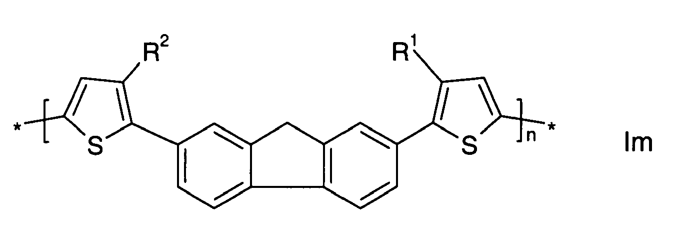 worin n, R¹ und R² die in Anspruch 1 angegebenen Bedeutungen besitzen.

9. Monomere nach mindestens einem der Ansprüche 1 bis 8, ausgewählt aus den folgenden Formeln 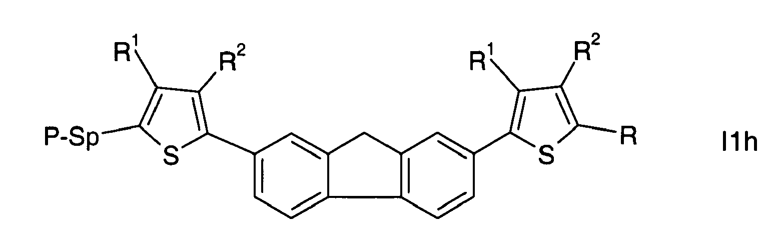 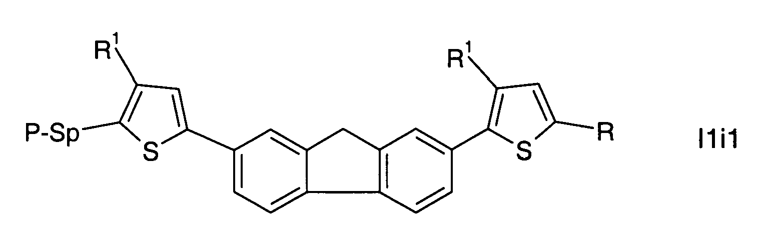 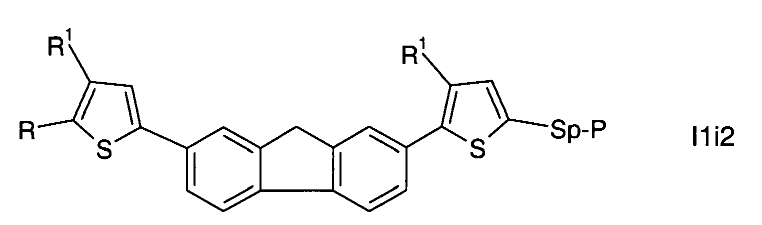 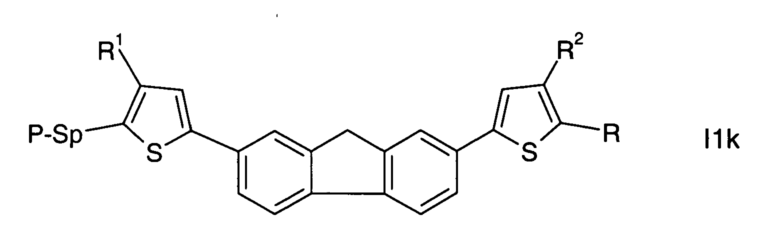 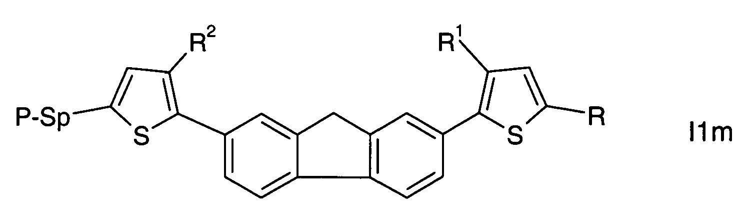 worin P, Sp, R¹ und R² wie in Anspruch 1 definiert sind und R eine der Bedeutungen von R⁷ in Anspruch 1 besitzt.

10. Monomere nach Anspruch 9, worin R P-Sp bedeutet.

11. Mono-, Oligo- oder Polymere nach mindestens einem der Ansprüche 1 bis 10, worin R⁷, R⁸ und R ausgewählt sind aus H, Halogen, Sn(R⁰)₃, CH₂Cl, COH, CH=CH₂, SiR⁰R⁰⁰ und gegebenenfalls substituiertem Aryl oder Heteroaryl.

12. Mono-, Oligo- oder Polymere nach mindestens einem der Ansprüche 1 bis 11, worin P ausgewählt ist aus CH₂=CW¹-COO-, 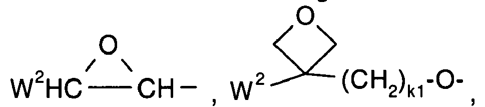 CH₂=CW²-(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN- und W⁴W⁵W⁶Si-, wobei W¹ H, Cl, CN, Phenyl oder Alkyl mit 1 bis 5 C-Atomen, insbesondere H, Cl oder CH₃, bedeutet, W² und W³ unabhängig voneinander H oder Alkyl mit 1 bis 5 C-Atomen, insbesondere Methyl, Ethyl oder n-Propyl, bedeuten, W⁴, W⁵ und W⁶ unabhängig voneinander Cl, Oxaalkyl oder Oxacarbonylalkyl mit 1 bis 5 C-Atomen bedeuten, Phe 1,4-Phenylen bedeutet, welches gegebenenfalls durch eine oder mehrere Gruppen R¹ wie in Anspruch 3 definiert substituiert ist, und k₁ und k₂ unabhängig voneinander 0 oder 1 bedeuten.

13. Polymerisierbares Flüssigkristallmaterial enthaltend ein oder mehrere Mono-, Oligo- oder Polymere nach mindestens einem der Ansprüche 1 bis 12 mit mindestens einer polymerisierbaren Gruppe und gegebenenfalls enthaltend eine oder mehrere weitere polymerisierbare Verbindungen, worin mindestens eines der polymerisierbaren Mono-, Oligo- und Polymere der Ansprüche 1 bis 12 und/oder der weiteren polymerisierbaren Verbindungen mesogen oder flüssigkristallin ist.

14. Anisotrope Polymerfolie mit Ladungstransporteigenschaften, erhältlich aus einem polymerisierbaren Flüssigkristallmaterial nach Anspruch 13, welches in seiner Flüssigkristallphase in makroskopisch uniforme Ausrichtung orientiert und zur Fixierung des ausgerichteten Zustandes polymerisiert oder vernetzt ist.

15. Seitenketten-Flüssigkristallpolymer erhalten durch Polymerisation eines oder mehrerer Mono- oder Oligomere oder eines polymerisierbaren Materials nach mindestens einem der Ansprüche 1 bis 13 oder durch Pfropfen eines oder mehrerer Mono- oder Oligomere oder eines polymerisierbaren Materials nach mindestens einem der Ansprüche 1 bis 13 in einer polymeranalogen Reaktion auf ein Polymerrückgrat, gegebenenfalls mit einem oder mehreren zusätzlichen mesogenen oder nicht mesogenen Comonomeren.

16. Verwendung der Mono-, Oligo- und Polymere, polymerisierbaren Materialien und Polymere nach mindestens einem der Ansprüche 1 bis 15 als Halbleiter oder Ladungstransportmaterialien in optischen, elektrooptischen oder elektronischen Vorrichtungen, wie Feldeffekttransistoren (FET) beispielsweise als Komponenten von integrierten Schaltungen, von Dünnfilmtransistoren (thin film transistors - TFT) für Flachbildschirm-Anwendungen oder von RFID-Tags (radio frequency identification tags) oder Halbleiterkomponenten für Anwendungen mit organischen Leuchtdioden (organic light emitting diode - OLED), darunter sowohl die Ladungstransport- als auch die Elektrolumineszenzschicht in Elektrolumineszenzanzeigen oder Hintergrundbeleuchtungen von Flüssigkristallanzeigen (liquid crystal displays - LCD).

17. Verwendung der Mono-, Oligo- und Polymere, polymerisierbaren Materialien und Polymere nach mindestens einem der Ansprüche 1 bis 15 als Elektrolumineszenzmaterial, in Photovoltaik- oder Sensorvorrichtungen, als Elektrodenmaterialien in Batterien, als Photoleiter, für elektrophotographische Anwendungen wie elektrophotographische Aufzeichnung und als Orientierungsschicht in LCD- oder OLED-Vorrichtungen.

18. Optische, elektrooptische oder elektronische Vorrichtung, FET, integrierte Schaltung (integrated circuit - IC), TFT, OLED oder Orientierungsschicht enthaltend ein Material, eine Komponente oder eine Vorrichtung nach mindestens einem der Ansprüche 1 bis 15.

19. TFT oder TFT-Anordnung für Flachbildschirme, RFID-Tag, Elektrolumineszenzanzeige oder Hintergrundbeleuchtung enthaltend ein Material, eine Komponente oder eine Vorrichtung nach mindestens einem der Ansprüche 1 bis 15 oder einen FET, eine IC, einen TFT oder eine OLED nach Anspruch 18.

20. Sicherheitsmarkierung oder -vorrichtung enthaltend einen FET oder ein RFID-Tag nach Anspruch 18 oder 19.

21. Mono-, Oligo- und Polymer, Material oder Polymer wie in mindestens einem der Ansprüche 1 bis 15 definiert, welches oxidativ oder reduktiv dotiert ist, so dass es leitende Ionenspezies bildet.

22. Ladungsinjektionsschicht, Planarisierungsschicht, Antistatikfolie oder leitendes Substrat oder leitende Struktur für Elektronikanwendungen oder Flachbildschirme, enthaltend ein Mono-, Oligo- oder Polymer, Material oder Polymer nach Anspruch 21.

## Revendications

1. Mono-, oligo- ou polymères choisis à partir de la formule I1 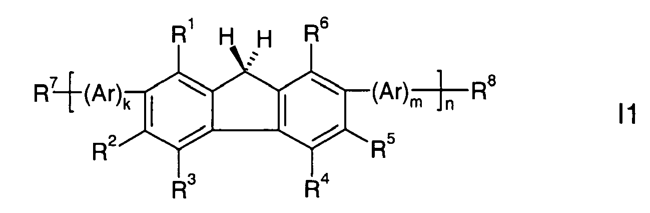 dans laquelle
R¹ à R⁶ sont indépendamment les uns des autres H, halogène ou alkyle en chaîne droite, ramifié ou cyclique avec 1 à 20 atomes de C, qui peut être non substitué, mono- ou polysubstitué par F, CI, Br, I et/ou CN, étant également possible pour un ou plusieurs groupes CH₂ non adjacents qu'ils soient remplacés, dans chaque cas indépendamment les uns des autres, par -O-, -S-, -NH-, -NR⁰-, -SiR⁰R⁰⁰-, -CO-, -COO-, -OCO-, -OCO-O-, -S-CO-, -CO-S-, -CH=CH- ou -C≡C- de telle sorte que des atomes de O et/ou de S ne soient pas directement liés à un autre, aryle ou hétéroaryle optionnellement substitué, ou P-Sp-,
P est un groupe polymérisable ou réactif,
Sp est un groupe espaceur ou une liaison simple,
R⁰ et R⁰⁰ sont indépendamment l'un de l'autre H ou alkyle avec 1 à 12 atomes de C,
Ar est un groupe arylène ou hétéroarylène qui est optionnellement substitué avec un ou plusieurs groupes R¹, et
k et m sont indépendamment l'un de l'autre 0, 1, 2, 3 ou 4, avec k+m > 1, ou k est 0 ou 1 et m est 1 ou 2,
R⁷ et R⁸ sont indépendamment l'un de l'autre H, halogène, Sn(R⁰)₃ ou alkyle en chaîne droite, ramifié ou cyclique avec 1 à 20 atomes de C, qui peut être non substitué, mono- ou polysubstitué par F, CI, Br, I, CN et/ou OH, étant également possible pour un ou plusieurs groupes CH₂ non adjacents qu'ils soient remplacés, dans chaque cas indépendam-ment les uns des autres, par -O-, -S-, -NH-, -NR⁰-, -SiR⁰R⁰⁰-, -CO-, -COO-, -OCO-, -OCO-O-, -S-CO-, -CO-S-, -CH=CH- ou -C≡C- de telle sorte que des atomes de O et/ou de S ne soient pas directement liés à un autre, aryle ou hétéroaryle optionnellement substitué, ou P-Sp,
n est un entier de 1 à 10000,
dans lesquels les unités récurrentes peuvent être identiques ou différentes,
dans lesquels
n est de 2 à 5000, ou
n est un entier de 1 à 15 et l'un de R⁷ et R⁸ ou les deux représentent P-Sp-, ou
R⁷ et R⁸ sont choisis parmi halogène, Sn(R⁰)₃, CH₂Cl, COH, CH=CH₂, SiR⁰R⁰⁰.

2. Oligo- ou polymères selon la revendication 1 avec un degré de polymérisation de 10 à 5000.

3. Oligo- ou polymères selon la revendication 1 ou 2 ayant des unités récurrentes identiques.

4. Mono-, oligo- ou polymères selon au moins l'une des revendications 1 à 3, dans lesquels R¹ à R⁶ sont H.

5. Mono-, oligo- ou polymères selon au moins l'une des revendications 1 à 4, dans lesquels R¹ à R⁶ sont choisis parmi C₁-C₂₀-alkyle qui est optionnellement substitué avec un ou plusieurs atomes de fluor, C₁-C₂₀-alkényle, C₁-C₂₀-alkynyle, C₁-C₂₀-alcoxy, C₁-C₂₀-thioéther, C₁-C₂₀-silyle, C₁-C₂₀-ester, C₁-C₂₀-amino, C₁-C₂₀-fluoroalkyle, (CH₂CH₂O)ₘCᵢH₂ᵢ₊₁, m étant de 1 à 6, i étant 1, 2 ou 3 et aryle ou hétéroaryle optionnellement substitué.

6. Mono-, oligo- ou polymères selon au moins l'une des revendications 1 à 5, dans lesquels Ar est choisi parmi phényle, phényle fluoré, pyridine, pyrimidine, biphényle, naphtalène, thiophène, thiophène fluoré, benzo[1,2-b:4,5-b']dithiophène, cyclopenta-[2,1-b:3,4-b']dithiophène, 9-alkylidènefluorène, 9-H,H-fluorène, thiazole et oxazole, tous ceux-ci sont non substitués, mono- ou polysubstitués avec R¹ comme défini dans la revendication 1 ou avec L, dans lequel L est F, CI, Br, ou un groupe alkyle, alcoxy, alkylcarbonyle ou alcoxycarbonyle avec 1 à 12 atomes de C, dans lesquels un ou plusieurs atomes de H sont optionnellement remplacés par F ou CI.

7. Mono-, oligo- ou polymères selon la revendication 6, dans lesquels Ar est thiophène-2,5-diyle qui est optionnellement substitué en position 3 ou 4 par R¹.

8. Mono-, oligo- ou polymères selon au moins l'une des revendications 1 à 7, choisis parmi les formules suivantes 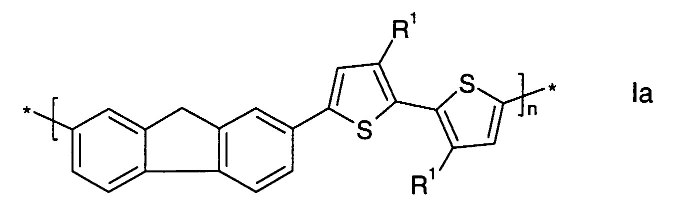 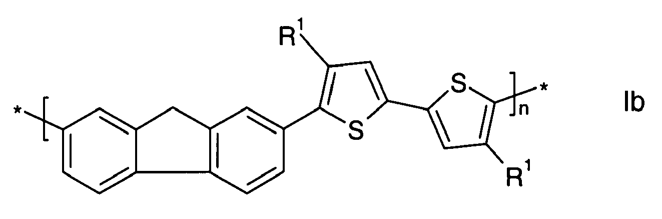 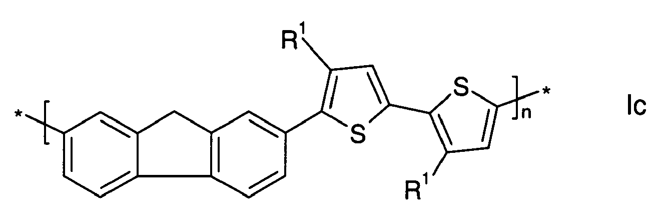 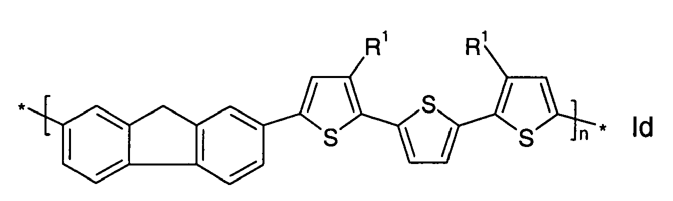 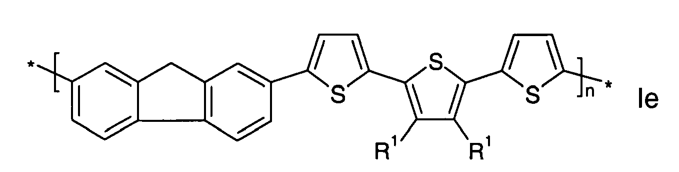 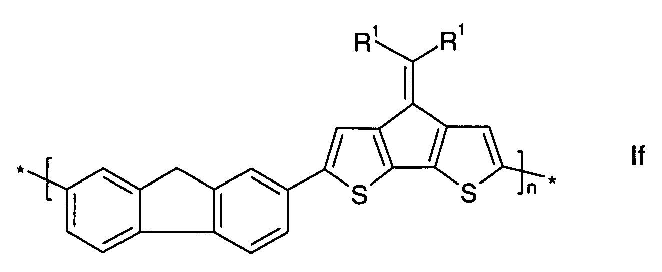 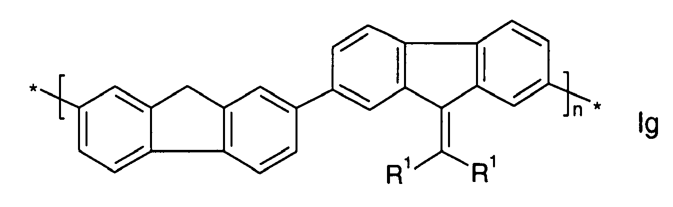 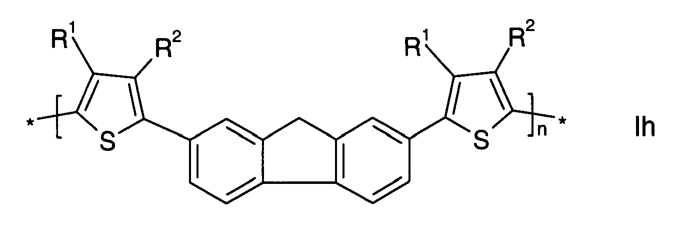 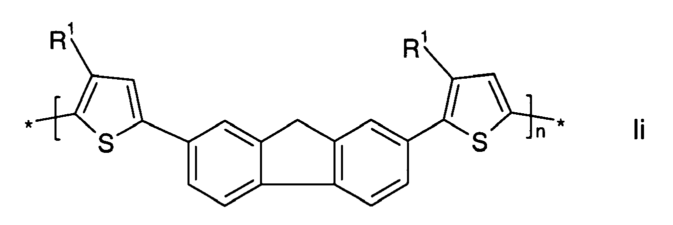 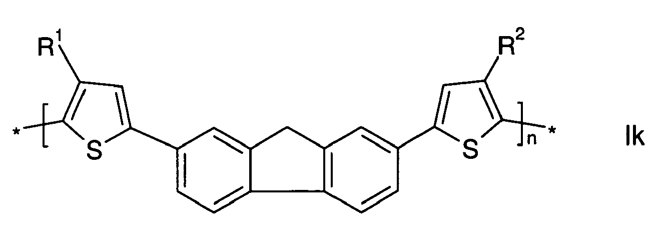 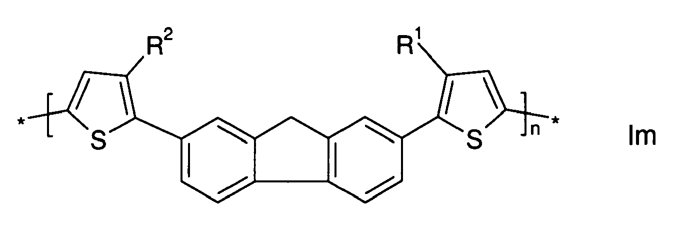 dans lesquelles n, R¹ et R² ont les significations données dans la revendication 1.

9. Monomères selon au moins l'une des revendications 1 à 8, choisis parmi les formules suivantes 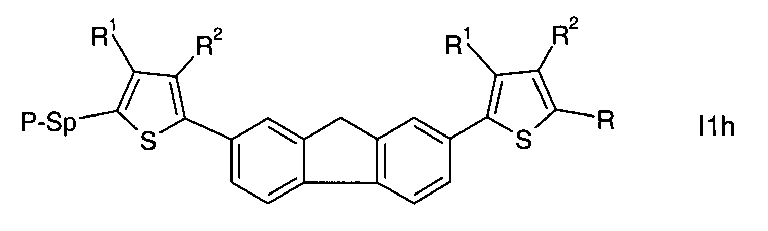 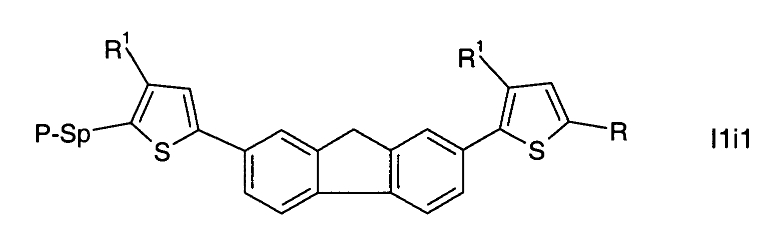 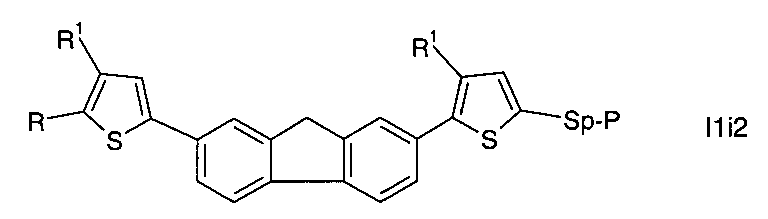 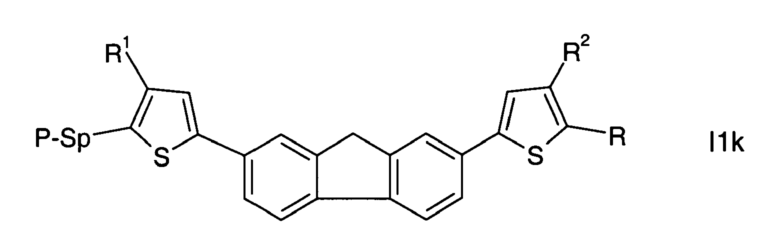 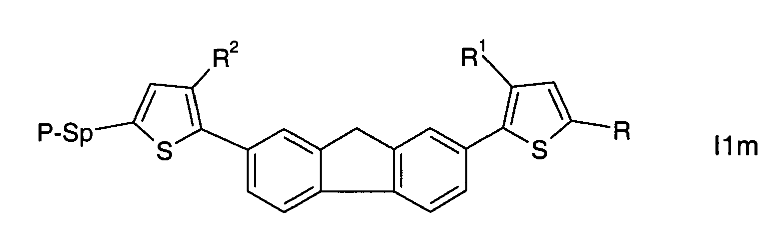 dans lesquelles P, Sp, R¹ et R² sont comme définis dans la revendication 1 et R a une des significations de R⁷ dans la revendication 1.

10. Monomères selon la revendication 9, dans lesquels R est P-Sp.

11. Mono-, oligo- ou polymères selon au moins l'une des revendications 1 à 10, dans lesquels R⁷, R⁸ et R sont choisis parmi H, halogène, Sn(R⁰)₃ , CH₂Cl, COH, CH=CH₂, SiR⁰R⁰⁰ et aryle ou hétéroaryle optionnellement substitué.

12. Mono-, oligo- ou polymères selon au moins l'une des revendications 1 à 11, dans lesquels P est choisi parmi CH₂=CW¹-COO-, 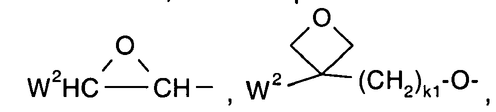 CH₂=CW²-(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN- et W⁴W⁵W⁶Si-, avec W¹ qui est H, CI, CN, phényle ou alkyle avec 1 à 5 atomes de C, en particulier H, Cl ou CH₃, W² et W³ étant indépendamment l'un de l'autre H ou alkyle avec 1 à 5 atomes de C, en particulier méthyle, éthyle ou n-propyle, W⁴, W⁵ et W⁶ étant indépendamment les uns des autres CI, oxaalkyle ou oxacarbonylalkyle avec 1 à 5 atomes de C, Phe étant 1,4-phénylène qui est optionnellement substitué par un ou plusieurs groupes R¹ comme défini dans la revendication 1, et k₁ et k₂ étant indépendamment l'un de l'autre 0 ou 1.

13. Matériau de cristal liquide polymérisable comprenant un ou plusieurs mono-, oligo- ou polymères selon au moins l'une des revendications 1 à 12 comprenant au moins un groupe polymérisable, et comprenant optionnellement un ou plusieurs composés polymérisables supplémentaires, dans lequel au moins un des mono-, oligo- et polymères polymérisables des revendications 1 à 12 et/ou des composés polymérisables supplémentaires est cristallin liquide ou mésogène.

14. Film polymère anisotropique avec des propriétés de transport de charges pouvant être obtenu à partir d'un matériau de cristal liquide polymérisable selon la revendication 13 qui est aligné dans sa phase de cristal liquide selon une orientation uniforme macroscopiquement et polymérisé ou réticulé pour fixer l'état orienté.

15. Polymère de cristal liquide à chaîne latérale pouvant être obtenu par polymérisation d'un ou plusieurs mono- ou oligomères ou un matériau polymérisable selon au moins l'une des revendications 1 à 13 ou en greffant un ou plusieurs mono- ou oligomères ou un matériau polymérisable selon au moins l'une des revendications 1 à 13 sur une ossature de polymère dans une réaction analogue à une polymérisation, optionnellement avec un ou plusieurs comonomères mésogènes ou non mésogènes additionnels.

16. Utilisation des mono-, oligo- et polymères, matériaux polymérisables et polymères selon au moins l'une des revendications 1 à 15 en tant que semiconducteurs ou matériaux de transport de charge dans des dispositifs optiques, électrooptiques ou électroniques, tels que des transistors à effet de champ (FET) par exemple en tant que composants de circuits intégrés, de transistors à films minces (TFT) pour des applications d'affichage à écran plat, ou d'étiquettes d'identification radio-fréquence (RFID), ou composants semiconducteurs pour des applications de diodes émettrices de lumière (OLED) incluant à la fois les couches de transport de charges et électroluminescentes dans des affichages électroluminescents ou des éclairages arrières d'affichages à cristaux liquides (LCD).

17. Utilisation des mono-, oligo- et polymères, matériaux polymérisables et polymères selon au moins l'une des revendications 1 à 15 en tant que matériau électroluminescent, dans des dispositifs photovoltaïques ou de capteurs, en tant que matériau d'électrode dans des batteries, en tant que photoconductor, pour des applications électrophotographiques telles que l'enregistrement électrophotographique et en tant que couche d'alignement dans des dispositifs LCD ou OLED.

18. Dispositif optique, électrooptique ou électronique, FET, circuit intégré (IC), TFT, OLED ou couche d'alignement comprenant un matériau, composant ou dispositif selon au moins l'une des revendications 1 à 16.

19. TFT ou réseau de TFTs pour affichages à écran plat, étiquette d'identification radio-fréquence (RFID), affichage ou éclairage arrière électroluminescent, comprenant un matériau, composant ou dispositif selon au moins l'une des revendications 1 à 15 ou un FET, IC, TFT ou OLED selon la revendication 18.

20. Marquage ou dispositif de sécurité comprenant un FET ou une étiquette RFID selon la revendication 18 ou 19.

21. Mono-, oligo- et polymère, matériau ou polymère comme défini dans au moins l'une des revendications 1 à 15, qui est dopé de façon oxydative ou réductive pour former des espèces ioniques conductrices.

22. Couche d'injection de charge, couche de planarisation, film antistatique ou substrat ou motif conducteur pour des applications électroniques ou pour des affichages à écran plat, comprenant un mono-, oligo- et polymère, matériau ou polymère selon la revendication 21.
